# EUROPEAN PATENT SPECIFICATION

(11) **EP 2 510 113 B1**
(45) Date of publication and mention of the grant of the patent: **08.02.2017**
(21) Application number: 10787761.5
(22) Date of filing: 07.12.2010
(51) Int. Cl.: C12Q 1/68

(54) **EGFR AND PTEN GENE ALTERATIONS PREDICTS SURVIVAL IN PATIENTS WITH BRAIN TUMORS**
EGFR- UND PTEN-GENVERÄNDERUNGEN ZUR VORHERSAGE DES ÜBERLEBENS VON PATIENTEN MIT HIRNTUMOREN
ALTÉRATIONS DES GÈNES EGFR ET PTEN POUR PRÉDIRE LA SURVIE DE PATIENTS ATTEINTS DE TUMEURS CÉRÉBRALES

(30) Priority: 07.12.2009 EP 09382271
(43) Date of publication of application: 17.10.2012
(73) Proprietor: Europath Biosciences, S.L., 08025 Barcelona (ES)
(72) Inventor: DONOVAN, Michael J., E- 08025 Barcelona (ES); COLOMER VALERO, Anna, E- 08025 Barcelona (ES); ERILL SAGALÉS, Nadina, E- 08025 Barcelona (ES); FERRER ABIZANDA, Isidre, E- 08907 Hospitalet de Llobregat, Barcelona (ES); BOLUDA CASAS, Susana, E- 08907 Hospitalet de Llobregat, Barcelona (ES)
(74) Representative: ABG Patentes, S.L.
(86) International application number: PCT/EP2010/069058
(87) International publication number: WO 2011/070005

(56) References cited:
- WO-A1-2010/028099
- WO-A2-2006/116016
- UMESH S ET AL: "Clinical and immunohistochemical prognostic factors in adult glioblastoma patients" CLINICAL NEUROPATHOLOGY, DUSTRI VERLAG, MUENCHEN-DEISENHOFEN, DE, vol. 28, no. 5, 1 January 2009 (2009-01-01), pages 362-372, XP009132032 ISSN: 0722-5091
- FELSBERG J ET AL: "Prognostic significance of molecular markers and extent of resection in primary glioblastoma patients" CLINICAL CANCER RESEARCH 20091101 AMERICAN ASSOCIATION FOR CANCER RESEARCH INC. USA LNKD- DOI:10.1158/1078-0432.CCR-08-2801, vol. 15, no. 21, 1 November 2009 (2009-11-01), pages 6683-6693, XP7912623 ISSN: 1078-0432
- VAN DEN BENT M J ET AL: "Randomized phase II trial of erlotinib versus temozolomide or carmustine in recurrent glioblastoma: EORTC brain tumor group study 26034" JOURNAL OF CLINICAL ONCOLOGY 20090310 US LNKD- DOI:10.1200/JCO.2008.17.5984, vol. 27, no. 8, 10 March 2009 (2009-03-10), pages 1268-1274, XP7912624 ISSN: 0732-183X
- SMITH JUSTIN S ET AL: "PTEN mutation, EGFR amplification, and outcome in patients with anaplastic astrocytoma and glioblastoma multiforme" JOURNAL OF THE NATIONAL CANCER INSTITUTE (BETHESDA), vol. 93, no. 16, 15 August 2001 (2001-08-15), pages 1246-1256, XP7912625 ISSN: 0027-8874
- ERMOIAN RALPH P ET AL: "Dysregulation of PTEN and protein kinase B is associated with glioma histology and patient survival." CLINICAL CANCER RESEARCH : AN OFFICIAL JOURNAL OF THE AMERICAN ASSOCIATION FOR CANCER RESEARCH MAY 2002 LNKD- PUBMED:12006525, vol. 8, no. 5, May 2002 (2002-05), pages 1100-1106, XP2526504 ISSN: 1078-0432
- MELLINGHOFF I K ET AL: "Molecular determinants of the response of glioblastomas to EGFR kinase inhibitors" NEW ENGLAND JOURNAL OF MEDICINE, MASSACHUSETTS MEDICAL SOCIETY, BOSTON, MA, US LNKD- DOI:10.1056/NEJMOA051918, vol. 353, 1 November 2005 (2005-11-01), pages 2012-2024, XP003005372 ISSN: 1533-4406
- NOBUSAWA SUMIHITO ET AL: "Intratumoral Patterns of Genomic Imbalance in Glioblastomas" BRAIN PATHOLOGY, vol. 20, no. 5, September 2010 (2010-09), pages 936-944, XP002623313
- KIM B ET AL: "The clinicopathologic values of the molecules associated with the main pathogenesis of the glioblastoma" JOURNAL OF NEUROLOGICAL SCIENCES, ELSEVIER SCIENTIFIC PUBLISHING CO, AMSTERDAM, NL, vol. 294, no. 1-2, 15 July 2010 (2010-07-15), pages 112-118, XP027084621 ISSN: 0022-510X [retrieved on 2010-05-02]

### SEQUENCE LISTING

<110> Europath Biosciences S.L.
<120> EGFR AND PTEN GENE ALTERNATIONS PREDICTS SURVIVAL IN PATIENTS WITH BRAIN TUMORS
<130> P5001PC00
<150> EP 09382271.6
   <151> 2009-12-07
<160> 13
<170> PatentIn version 3.5
<210> 1
   <211> 5572
   <212> DNA
   <213> Homo sapiens
<400> 1
   cctcccctcg cccggcgcgg tcccgtccgc ctctcgctcg cctcccgcct cccctcggtc      60
   ttccgaggcg cccgggctcc cggcgcggcg gcggaggggg cgggcaggcc ggcgggcggt     120
   gatgtggcgg gactctttat gcgctgcggc aggatacgcg ctcggcgctg ggacgcgact     180
   gcgctcagtt ctctcctctc ggaagctgca gccatgatgg aagtttgaga gttgagccgc     240
   tgtgaggcga ggccgggctc aggcgaggga gatgagagac ggcggcggcc gcggcccgga     300
   gcccctctca gcgcctgtga gcagccgcgg gggcagcgcc ctcggggagc cggccggcct     360
   gcggcggcgg cagcggcggc gtttctcgcc tcctcttcgt cttttctaac cgtgcagcct     420
   cttcctcggc ttctcctgaa agggaaggtg gaagccgtgg gctcgggcgg gagccggctg     480
   aggcgcggcg gcggcggcgg cacctcccgc tcctggagcg ggggggagaa gcggcggcgg      540
   cggcggccgc ggcggctgca gctccaggga gggggtctga gtcgcctgtc accatttcca     600
   gggctgggaa cgccggagag ttggtctctc cccttctact gcctccaaca cggcggcggc     660
   ggcggcggca catccaggga cccgggccgg ttttaaacct cccgtccgcc gccgccgcac     720
   cccccgtggc ccgggctccg gaggccgccg gcggaggcag ccgttcggag gattattcgt     780
   cttctcccca ttccgctgcc gccgctgcca ggcctctggc tgctgaggag aagcaggccc     840
   agtcgctgca accatccagc agccgccgca gcagccatta cccggctgcg gtccagagcc     900
   aagcggcggc agagcgaggg gcatcagcta ccgccaagtc cagagccatt tccatcctgc     960
   agaagaagcc ccgccaccag cagcttctgc catctctctc ctcctttttc ttcagccaca    1020
   ggctcccaga catgacagcc atcatcaaag agatcgttag cagaaacaaa aggagatatc    1080
   aagaggatgg attcgactta gacttgacct atatttatcc aaacattatt gctatgggat    1140
   ttcctgcaga aagacttgaa ggcgtataca ggaacaatat tgatgatgta gtaaggtttt    1200
   tggattcaaa gcataaaaac cattacaaga tatacaatct ttgtgctgaa agacattatg    1260
   acaccgccaa atttaattgc agagttgcac aatatccttt tgaagaccat aacccaccac    1320
   agctagaact tatcaaaccc ttttgtgaag atcttgacca atggctaagt gaagatgaca 1380
   atcatgttgc agcaattcac tgtaaagctg gaaagggacg aactggtgta atgatatgtg 1440
   catatttatt acatcggggc aaatttttaa aggcacaaga ggccctagat ttctatgggg 1500
   aagtaaggac cagagacaaa aagggagtaa ctattcccag tcagaggcgc tatgtgtatt 1560
   attatagcta cctgttaaag aatcatctgg attatagacc agtggcactg ttgtttcaca 1620
   agatgatgtt tgaaactatt ccaatgttca gtggcggaac ttgcaatcct cagtttgtgg 1680
   tctgccagct aaaggtgaag atatattcct ccaattcagg acccacacga cgggaagaca 1740
   agttcatgta ctttgagttc cctcagccgt tacctgtgtg tggtgatatc aaagtagagt 1800
   tcttccacaa acagaacaag atgctaaaaa aggacaaaat gtttcacttt tgggtaaata 1860
   cattcttcat accaggacca gaggaaacct cagaaaaagt agaaaatgga agtctatgtg 1920
   atcaagaaat cgatagcatt tgcagtatag agcgtgcaga taatgacaag gaatatctag 1980
   tacttacttt aacaaaaaat gatcttgaca aagcaaataa agacaaagcc aaccgatact 2040
   tttctccaaa ttttaaggtg aagctgtact tcacaaaaac agtagaggag ccgtcaaatc 2100
   cagaggctag cagttcaact tctgtaacac cagatgttag tgacaatgaa cctgatcatt 2160
   atagatattc tgacaccact gactctgatc cagagaatga accttttgat gaagatcagc 2220
   atacacaaat tacaaaagtc tgaatttttt tttatcaaga gggataaaac accatgaaaa 2280
   taaacttgaa taaactgaaa atggaccttt ttttttttaa tggcaatagg acattgtgtc 2340
   agattaccag ttataggaac aattctcttt tcctgaccaa tcttgtttta ccctatacat 2400
   ccacagggtt ttgacacttg ttgtccagtt gaaaaaaggt tgtgtagctg tgtcatgtat 2460
   ataccttttt gtgtcaaaag gacatttaaa attcaattag gattaataaa gatggcactt 2520
   tcccgtttta ttccagtttt ataaaaagtg gagacagact gatgtgtata cgtaggaatt 2580
   ttttcctttt gtgttctgtc accaactgaa gtggctaaag agctttgtga tatactggtt 2640
   cacatcctac ccctttgcac ttgtggcaac agataagttt gcagttggct aagagaggtt 2700
   tccgaagggt tttgctacat tctaatgcat gtattcgggt taggggaatg gagggaatgc 2760
   tcagaaagga aataatttta tgctggactc tggaccatat accatctcca gctatttaca 2820
   cacacctttc tttagcatgc tacagttatt aatctggaca ttcgaggaat tggccgctgt 2880
   cactgcttgt tgtttgcgca ttttttttta aagcatattg gtgctagaaa aggcagctaa 2940
   aggaagtgaa tctgtattgg ggtacaggaa tgaaccttct gcaacatctt aagatccaca 3000
   aatgaaggga tataaaaata atgtcatagg taagaaacac agcaacaatg acttaaccat 3060
   ataaatgtgg aggctatcaa caaagaatgg gcttgaaaca ttataaaaat tgacaatgat 3120
   ttattaaata tgttttctca attgtaacga cttctccatc tcctgtgtaa tcaaggccag 3180
   tgctaaaatt cagatgctgt tagtacctac atcagtcaac aacttacact tattttacta 3240
   gttttcaatc ataatacctg ctgtggatgc ttcatgtgct gcctgcaagc ttcttttttc 3300
   tcattaaata taaaatattt tgtaatgctg cacagaaatt ttcaatttga gattctacag 3360
   taagcgtttt ttttctttga agatttatga tgcacttatt caatagctgt cagccgttcc 3420
   acccttttga ccttacacat tctattacaa tgaattttgc agttttgcac attttttaaa 3480
   tgtcattaac tgttagggaa ttttacttga atactgaata catataatgt ttatattaaa 3540
   aaggacattt gtgttaaaaa ggaaattaga gttgcagtaa actttcaatg ctgcacacaa 3600
   aaaaaagaca tttgattttt cagtagaaat tgtcctacat gtgctttatt gatttgctat 3660
   tgaaagaata gggttttttt tttttttttt tttttttttt ttaaatgtgc agtgttgaat 3720
   catttcttca tagtgctccc ccgagttggg actagggctt caatttcact tcttaaaaaa 3780
   aatcatcata tatttgatat gcccagactg catacgattt taagcggagt acaactacta 3840
   ttgtaaagct aatgtgaaga tattattaaa aaggtttttt tttccagaaa tttggtgtct 3900
   tcaaattata ccttcacctt gacatttgaa tatccagcca ttttgtttct taatggtata 3960
   aaattccatt ttcaataact tattggtgct gaaattgttc actagctgtg gtctgaccta 4020
   gttaatttac aaatacagat tgaataggac ctactagagc agcatttata gagtttgatg 4080
   gcaaatagat taggcagaac ttcatctaaa atattcttag taaataatgt tgacacgttt 4140
   tccatacctt gtcagtttca ttcaacaatt tttaaatttt taacaaagct cttaggattt 4200
   acacatttat atttaaacat tgatatatag agtattgatt gattgctcat aagttaaatt 4260
   ggtaaagtta gagacaacta ttctaacacc tcaccattga aatttatatg ccaccttgtc 4320
   tttcataaaa gctgaaaatt gttacctaaa atgaaaatca acttcatgtt ttgaagatag 4380
   ttataaatat tgttctttgt tacaatttcg ggcaccgcat attaaaacgt aactttattg 4440
   ttccaatatg taacatggag ggccaggtca taaataatga cattataatg ggcttttgca 4500
   ctgttattat ttttcctttg gaatgtgaag gtctgaatga gggttttgat tttgaatgtt 4560
   tcaatgtttt tgagaagcct tgcttacatt ttatggtgta gtcattggaa atggaaaaat 4620
   ggcattatat atattatata tataaatata tattatacat actctcctta ctttatttca 4680
   gttaccatcc ccatagaatt tgacaagaat tgctatgact gaaaggtttt cgagtcctaa 4740
   ttaaaacttt atttatggca gtattcataa ttagcctgaa atgcattctg taggtaatct 4800
   ctgagtttct ggaatatttt cttagacttt ttggatgtgc agcagcttac atgtctgaag 4860
   ttacttgaag gcatcacttt taagaaagct tacagttggg ccctgtacca tcccaagtcc 4920
   tttgtagctc ctcttgaaca tgtttgccat acttttaaaa gggtagttga ataaatagca 4980
   tcaccattct ttgctgtggc acaggttata aacttaagtg gagtttaccg gcagcatcaa 5040
   atgtttcagc tttaaaaaat aaaagtaggg tacaagttta atgtttagtt ctagaaattt 5100
   tgtgcaatat gttcataacg atggctgtgg ttgccacaaa gtgcctcgtt tacctttaaa 5160
   tactgttaat gtgtcatgca tgcagatgga aggggtggaa ctgtgcacta aagtgggggc 5220
   tttaactgta gtatttggca gagttgcctt ctacctgcca gttcaaaagt tcaacctgtt 5280
   ttcatataga atatatatac taaaaaattt cagtctgtta aacagcctta ctctgattca 5340
   gcctcttcag atactcttgt gctgtgcagc agtggctctg tgtgtaaatg ctatgcactg 5400
   aggatacaca aaaataccaa tatgatgtgt acaggataat gcctcatccc aatcagatgt 5460
   ccatttgtta ttgtgtttgt taacaaccct ttatctctta gtgttataaa ctccacttaa 5520
   aactgattaa agtctcattc ttgtcaaaaa aaaaaaaaaa aaaaaaaaaa aa 5572
<210> 2
   <211> 403
   <212> PRT
   <213> Homo sapiens
<400> 2
<210> 3
   <211> 8229
   <212> DNA
   <213> Mus musculus
<400> 3
   gcggcaggat acgcgcttgg gcgtcgggac gcggctgcgc tcagctctct cctctcggaa 60
   gctgcagcca tgatggaagt ttgagagttg agccgctgtg aggccaggcc cggcgcaggc 120
   gagggagatg agagacggcg gcggccacgg cccagagccc ctctcagcgc ctgtgagcag 180
   ccgcgggggc agcgccctcg gggagccggc cgggcggcgg cggcggcagc ggcggcgggc 240
   ctcgcctcct cgtcgtctgt tctaaccggg cagcttctga gcagcttcgg agagagacgg 300
   tggaagaagc cgtgggctcg agcgggagcc ggcgcaggct cggcggctgc acctcccgct 360
   cctggagcgg gggggagaag cggcggcggc ggccgcggct ccggggaggg ggtcggagtc 420
   gcctgtcacc attgccaggg ctgggaacgc cggagagttg ctctctcccc ttctcctgcc 480
   tccaacacgg cggcggcggc ggcggcacgt ccagggaccc gggccggtgt taagcctccc 540
   gtccgccgcc gccgcacccc ccctggcccg ggctccggag gccgccggag gaggcagccg 600
   ctgcgaggat tatccgtctt ctccccattc cgctgcctcg gctgccaggc ctctggctgc 660
   tgaggagaag caggcccagt ctctgcaacc atccagcagc cgccgcagca gccattaccc 720
   ggctgcggtc cagggccaag cggcagcaga gcgaggggca tcagcgaccg ccaagtccag 780
   agccatttcc atcctgcaga agaagcctcg ccaccagcag cttctgccat ctctctcctc 840
   ctttttcttc agccacaggc tcccagacat gacagccatc atcaaagaga tcgttagcag 900
   aaacaaaagg agatatcaag aggatggatt cgacttagac ttgacctata tttatccaaa 960
   tattattgct atgggatttc ctgcagaaag acttgaaggt gtatacagga acaatattga 1020
   tgatgtagta aggtttttgg attcaaagca taaaaaccat tacaagatat acaatctatg 1080
   tgctgagaga cattatgaca ccgccaaatt taactgcaga gttgcacagt atccttttga 1140
   agaccataac ccaccacagc tagaacttat caaacccttc tgtgaagatc ttgaccaatg 1200
   gctaagtgaa gatgacaatc atgttgcagc aattcactgt aaagctggaa agggacggac 1260
   tggtgtaatg atttgtgcat atttattgca tcggggcaaa tttttaaagg cacaagaggc 1320
   cctagatttt tatggggaag taaggaccag agacaaaaag ggagtcacaa ttcccagtca 1380
   gaggcgctat gtatattatt atagctacct gctaaaaaat cacctggatt acagacccgt 1440
   ggcactgctg tttcacaaga tgatgtttga aactattcca atgttcagtg gcggaacttg 1500
   caatcctcag tttgtggtct gccagctaaa ggtgaagata tattcctcca attcaggacc 1560
   cacgcggcgg gaggacaagt tcatgtactt tgagttccct cagccattgc ctgtgtgtgg 1620
   tgatatcaaa gtagagttct tccacaaaca gaacaagatg ctcaaaaagg acaaaatgtt 1680
   tcacttttgg gtaaatacgt tcttcatacc aggaccagag gaaacctcag aaaaagtgga 1740
   aaatggaagt ctttgtgatc aggaaatcga tagcatttgc agtatagagc gtgcagataa 1800
   tgacaaggag tatcttgtac tcaccctaac aaaaaacgat cttgacaaag caaacaaaga 1860
   caaggccaac cgatacttct ctccaaattt taaggtgaaa ctatacttta caaaaacagt 1920
   agaggagcca tcaaatccag aggctagcag ttcaacttct gtgactccag atgttagtga 1980
   caatgaacct gatcattata gatattctga caccactgac tctgatccag agaatgaacc 2040
   ttttgatgaa gatcagcatt cacaaattac aaaagtctga tttttttttt cttatcaaga 2100
   gggataaaat accatgaaaa aaaaaaaact tgaataaact gaaatggacc tttttttttt 2160
   tttttttttt ttaaatggca ataggacatt gtgtcagatt gcagttatag gaacaattct 2220
   cttctcctga ccaatcttgt tttaccctat acatccacag ggttttgaca cttgttgtcc 2280
   agttaaaaaa aggttgtgta gctgtgtcat gtatatacct ttttgtgtca aaaggacatt 2340
   taaaattcaa ttaggataaa taaaagatgg cactttccca ttttattcca gttttataaa 2400
   aagtggagac aggctgatgt gtatacgcag gagtttttcc tttattttct gtcaccagct 2460
   gaagtggctg aagagctctg attcccgggt tcacgtccta cccctttgca cttgtggcaa 2520
   cagataagtt tgcagttggc taaggaagtt tctgcagggt tttgttagat tctaatgcat 2580
   gcacttgggt tgggaatgga gggaatgctc agaaaggaat gtttctacct gggctctgga 2640
   ccatacacca tctccagctc cttagatgca cctttcttta gcatgctcca cttactaatc 2700
   tggacatccg agagattggc tgctgtcctg ctgtttgttt gtgcatttta aagagcatat 2760
   tggtgctaga caaggcagct agagtgagta tatttgtagt ggggtacagg aatgaaccat 2820
   ctacagcatc ttaagaatcc acaaaggaag ggatataaaa aaagtggtca tagatagata 2880
   aaagacacag cagcaatgac ttaaccatac aaatgtggag gctttcaaca aaggatgggc 2940
   tggaaacaga aaatttgaca atgatttatt cagtatgctt tctcagttgt aatgactgct 3000
   ccatctccta tgtaatcaag gccagtgcta agagtcagat gctattagtc cctacatcag 3060
   tcaacacctt acctttattt ttattaattt tcaatcatat acctactgtg gatgcttcat 3120
   gtgctggctg ccagtttgtt tttctcctta aatattttat aattcttcac aggaaatttc 3180
   aacttgagat tcaacagtaa gcaggttttg tttttttttt ttcctagaga ttgatgatgc 3240
   gcgtcctcag tccagtggct gtcagacgtt cagccccttt gaccttacac attctattac 3300
   aatgagtttt gcagttttgc acattttttt taaatgtcat taactgttag ggaattttac 3360
   ttgaatactg aatacatata atgtgtatat taaaaaagtc attgtttgtg ttaaaaaaga 3420
   aattagagtt gcagtaaatt tacagcactg cacgaataat aaggcattga agtttttcag 3480
   tagaaattgt cctacagatg ctttatcgac ttgctattgg aagaatagat cttcttaaat 3540
   gtgcagtgtt gagtcacttc gttatagtgg tagagttggg attagggctt caattttact 3600
   tcttaaatat cattctatgt ttgatatgcc cagactgcat acaatttaaa gcaagagtac 3660
   aactactatc gtaatggtaa tgtgaagatg ctattacaaa ggatctcctc ccaacccctc 3720
   gggaatttgg tgtctttcaa attatatctt gaccttgaca tttgaatatc cagccattat 3780
   tagatttctt aatggtgtga agtcccattt tcaataactt attggtgctg aaattgttca 3840
   ctagctgtgg tctgacctag ttaatttaca agtacagatt gcataggacc cactagagaa 3900
   gcatttatag tttgatggta agtagattag gcagaacgcc atctaaaata ttcttagaaa 3960
   ataatgttga tgtattttcc atacctcatc agtttcactc aaccaataaa gtttttaaaa 4020
   ttgtaacaaa gctcttagga tttacacatt tatatttaaa cattgataca tgaatattga 4080
   ctgactgttg ataaagtcag agacaacttt tcctgagatc tcaccatgga aatctgtaca 4140
   cccccttgtc tttcctaaaa gctgaaagtg gctgactaaa atgcaaagca gctgttgatg 4200
   ttttgaagat agtgataaac actgttcttt gttagttttg ggcacagcat gctaaactat 4260
   aacttgtatt gttccaatat gtaacacaga gggccaggtc atgaataatg acattacaat 4320
   gggctgttgc actgttaata tttttccttt ggaatgtgaa ggtctgaatg agggttttga 4380
   ttttgaatgt ttcagtgttt ttgagaagcc ttgcttacat tttatggtgt agtcattgga 4440
   aatggaaaaa tggcattata tatatattat atatatataa atatatatat tatacatact 4500
   ctccttactt tatttcagtt accatcccca tagaatttga caagaattgc tatgactgaa 4560
   agggttttga gtcctaattc aaactttctt tatgacagta ttcacgatta gcctgaagtg 4620
   cattctgtag gtgatctctc ccgtgtttct ggaatgcttt cttagactct tggatgtgca 4680
   gcagcttatg tgtctgaaat gacttgaagg catcaccttt aagaaggctt acagttgggc 4740
   cccgtacatc ccaagtcctc tgtaattcct cttggacatt tttgccataa ttgtaaaagg 4800
   gtagttgaat taaatagcgt caccattctt tgctgtggca caggttataa acttaagtgg 4860
   agtttaccgg cagcatcaaa tgtttcagct ttaaaaataa aagtaggtta caagttacat 4920
   gtttagtttt agaaaatttg tgcaatatgt tcataacgat ggctgtggtt gccacaaagt 4980
   gcctcgttta cctttaaata ctgttaatgt gtcgtgcatg cagacggaag gggtggatct 5040
   gtgcactaaa cggggggctt ttactctagt attcggcaga gttgccttct acctgccagc 5100
   tcaaaagttc gatctgtttt catatagaat atatatacta aaaccatcca gtctgtaaaa 5160
   cagccttacc ccgattcagc ctcttcagat actcttgtgc tgtgcagcag tggctctgtg 5220
   tgtaaatgct atgcactgag gatacacaaa tatgacgtgt acaggataat gcctcatacc 5280
   aatcagatgt ccatttgtta ctgtgtttgt taacaaccct ttatctctta gtgttataaa 5340
   ctccacttaa aactgattaa agtctcattc ttgtcattgt gtgggtgttt tattaaatga 5400
   gagtatttat aattcaaatt gcttaaatcc attaaaatgt tcagtaatgg gcagccacat 5460
   atgattacaa agttcctgtg catttttcta tttttccccc tccttgctat ccttccaagc 5520
   aaagcatctt tctgtcatct tggtagacac atacctgtct actcatggtt aagaagagca 5580
   ctttaagcct tagtcatcac ttaataagtt attccaggca cagtaaaaag ttcaaggttc 5640
   ttggaaaacg gtgcttattt ctcttcttat aagccagatg tctgaagata gccctaaccc 5700
   caagaacggg cttgatgtct caggtctgtt ctgtggcttt ctgttttttt taacactgca 5760
   gttggccatc agcacatggg aggtttcatc gggacttgtc cagagtagta ggctcaaata 5820
   tactatctcc tttctaatat tcttaaaggc taaggagtcc tttcaatata acagtaagat 5880
   aacttgtgat gttttagaag taagcagacc attaatgtca atgtggagtc ttaatgttac 5940
   atgaagttga tagtttctct gtgacccatt taaaaataca aaccgagtag catgcaatta 6000
   tgtaaagaaa tatgaagatt atatgtagtc acacattttc tttagaattc ttagtttggt 6060
   gaaaacttga atataaaggt attttgattt atatgacatt ttgatgatat ttgaaaaaaa 6120
   ggaatttcct gacattttgc ttttagatca tgtcccccat tgtgctgtaa tttaagccaa 6180
   cttggttcag tgaatgccat caccatttcc attgagaatt taaaactcac cagtgtttaa 6240
   catgcaggct tctgagggct cccggagaat cagaccttaa gcccagttga tttacttcta 6300
   acgtgaaact tcgagttcct gtatactttg ctagataatt tgtggtacat ctaaagctta 6360
   gtcttaagtg gcttgtgtgt ggattttatt caacattctt gttgctaggg tagagagaaa 6420
   tgttgctgag tagaaacaag agtacccagt tcaatgtggt acagagagca gtccctaaaa 6480
   tctgtacaca gtgtaatgga ccactttagg agtcaagagg ctgatttttc ctatgaaatt 6540
   acattgcaac aggaagcctt ctagtatagt tccttttact gttagaatat gtttttatgc 6600
   atacgctata gctgctttcc catcttccaa caacaggtat caggatgtaa gcaagcttta 6660
   aacagtgtga agatggcagg atagtgtcat cggtaacagt cctctgactc taaatgtagt 6720
   tgctctgtaa cactttgtga atataacatc acaattctca tgtccttggg gggggggggc 6780
   atacccagta ttagtatgtt ttagtgacta agcaatcatt tttctgttta ctcatgtaca 6840
   ttttctcttt aaaactaaaa cctgtactgt gtatgtctcc aaagcctttt agcttagttt 6900
   ttaggaaatg aacactgaat ggatcacttt ttagtgtagc aggtatggga tatgtgcatt 6960
   atagagagac cttgtcagct ctctgggcct atttgaatgt ttattgttgg tgtgaggatg 7020
   gtaggggaat cagtaaatac aagttacgtt ggtttagcag agcaagctca gtgtgggtat 7080
   ttctctttga agcgtggtgc gtgacgcact gtgagtagag aatttggtca ccctttgagt 7140
   cctcttgcat tttgcaaact tgctcagcaa atgcgtacct accttgcccc ctaggtaaaa 7200
   gcaggaacta ctactgattt atctgtcact cagctgtctt tatatgtgtg cttctgtgac 7260
   ttgtatcaca caagaatctt aaagatttca caaattgtta ccttttagct ctgaatgttg 7320
   agtattctgg tgggctaaca acaagacaaa ctcttgacag tcatttgaga attttcatga 7380
   aacatttagc tgaaaacatt ttataattta tgaaaaaaat gtgttacctt aaacttttac 7440
   atatgtggga gacattaact gccatatttg agcatactga attttaaatt taaaataaag 7500
   ctgcatattt ttaaatgaaa tgtttaacaa ggattcatat tttttgtttt ttaagattaa 7560
   aaataattta tgtcttctca tgtggaacct catctgtcac aatggttaga ttatacagaa 7620
   tggagcaagg cttgtagtgg tttagcttac agtaaaattc ttaatgttta gatgtgttta 7680
   cttactggct gttatgtata cttttgagat tttccacctg ttctgtgtag ttttctaaat 7740
   gatactccta cttaaaaaca gcattttagt atctattttc tgtctccatt aaatggtcct 7800
   cattttctat tgagtttgga agtgtgcaca ttgtgtgtgt gtgtgtgtgt gtgtgtgtgc 7860
   acacgtgtgc gcgcccgtgc gtgtgtctat ttgtggagtt tgtatgggag aattagtttt 7920
   gaaagtgcta gaatagagat gaaatttggt tcaagtaaaa ttttcccact gggattttac 7980
   agtttattgt aataaaatgt taattttgga tgaccttgaa tattaatgaa tttgttagcc 8040
   tcttgatgtg tgcattaatg agatatatca aagttgtata ttaaaccaaa gttggagttg 8100
   tggaagtgtt tttatgaagt tccgtttggc taccaatgga cataagacta gaaatacctt 8160
   cctgtggaga atatttttcc tttaaacaat taaaaaggtt cattattttt gaaaaaaaaa 8220
   aaaaaaaaa 8229
<210> 4
   <211> 1212
   <212> DNA
   <213> Rattus norvegicus
<400> 4
   atgacagcca tcatcaaaga gatcgttagc agaaacaaaa ggagatatca agaggatgga      60
   ttcgacttag acttgaccta tatttatcca aatattattg ctatgggatt tcctgcagaa     120
   agacttgaag gtgtatacag gaacaatatt gatgatgtag taaggttttt ggattcaaag     180
   cataaaaacc attacaagat atacaatcta tgtgctgaga gacattatga caccgccaaa     240
   tttaactgca gagttgcaca gtatcctttt gaagaccata acccaccaca gctagaactt     300
   atcaaaccct tttgtgaaga tcttgaccaa tggctaagtg aagacgacaa tcatgttgca     360
   gcaattcact gtaaagctgg gaaaggacgg actggtgtaa tgatttgtgc atatttattg     420
   catcggggca agtttttaaa ggcacaagag gccctggatt tttatgggga agtaaggacc     480
   agagataaaa agggagtaac tattcccagt cagaggcgct atgtatatta ttatagctac     540
   ctgttaaaga atcacctgga ttacagacca gtggcactgt tgtttcacaa gatgatgttt     600
   gaaactattc caatgttcag tggcggaact tgcaatcccc agtttgtggt ctgccagcta     660
   aaggtgaaga tctactcctc caactcagga cccacgcggc gggaggacaa gctcatgtac     720
   tttgagttcc ctcagccatt gcctgtgtgt ggtgacatca aagtagagtt cttccacaaa     780
   cagaacaaga tgctcaaaaa ggacaaaatg tttcactttt gggtaaatac gttcttcata     840
   ccaggaccag aggaaacctc agaaaaagtg gaaaatggaa gtctttgtga tcaggaaatc     900
   gatagcattt gtagtataga gcgtgcggat aatgacaagg agtatcttgt gctcaccctg     960
   acaaaaaatg atcttgacaa agcaaacaaa gacaaggcca accgatactt ctctccaaat    1020
   tttaaggtga agttatactt cacaaaaaca gtagaggagc catcaaatcc agaggctagc    1080
   agttcaactt ctgtgactcc agacgttagt gacaatgaac ctgatcatta tagatattct    1140
   gacaccactg actctgatcc agagaatgaa ccttttgatg aagatcagca ttcacaaatt    1200
   acaaaagtct ga                                                        1212
<210> 5
   <211> 1396
   <212> DNA
   <213> Canis familiaris
<400> 5
   ccgcccgccg ccaggcccgg ggccgcctgc agcctgcgga ggaggccgcg ccgcccgccg      60
   ctcctgccgt ctctctcctc cttcctctcc agccaccggc tcccagacat gacagccatc     120
   atcaaggaga tcgtcagcag aaacaaaagg cgctaccagg aggatgggtt cgacttggac     180
   ttgacctata tttatcccaa cattattgct atggggtttc ctgcagaaag acttgaaggc     240
   gtatacagga acaatattga tgatgtagta aggtttttgg attcaaagca taaaaaccat     300
   tacaagatat acaatctgtg tgctgaaaga cattatgata ccgccaaatt taactgcaga     360
   gttgcacagt atccttttga agaccataat ccaccacagc tagaacttat caaacccttt      420
   tgtgaagatc ttgaccaatg gctaagtgaa gatgacaatc atgttgcagc aattcactgt     480
   aaagctggaa agggacgaac tggtgtaatg atttgtgcat atttattaca tcggggcaaa     540
   tttctaaagg cacaagaggc cctagatttc tatggggaag taaggaccag agacaaaaag     600
   ggagtaacta ttcccagtca gaggcgctat gtgtattatt atagctacct gttaaagaat     660
   catctggatt atagaccagt ggcactgttg tttcacaaga tgatgtttga aactattcca     720
   atgttcagtg gcggaacttg caatcctcag tttgtggtct gccagctaaa ggtgaagatc     780
   tattcctcca attcaggacc cacacgacgg gaagacaagt tcatgtactt tgagttccct     840
   cagccattgc ctgtgtgcgg tgacatcaaa gtagagttct tccacaaaca gaacaagatg     900
   ctaaaaaagg acaaaatgtt tcacttttgg gtaaacacat tcttcatacc aggaccagag     960
   gaaacctcag aaaaagtaga aaatggaagt ctatgtgatc aagaaattga tagtatttgc    1020
   agtatagaac gtgcagataa tgacaaggaa tatctagtac tcactttaac aaaaaatgat    1080
   ctcgacaaag caaataaaga caaggccaac cgatattttt ctccaaattt taaggtgaag    1140
   ctgtacttca caaaaactgt agaggagcca tcaaacccgg aggctagcag ttcaacttct    1200
   gtgacgccag atgttagtga caatgaacct gatcattata gatattctga caccactgac    1260
   tctgacccag agaatgaacc ctttgatgaa gatcagcaca cacaaatcac aaaagtctga    1320
   atttttttta atcaagaggg ataaaacacc atgaaaacaa acttgaataa actgaaattg    1380
   gacctttttt ttttaa                                                    1396
<210> 6
   <211> 5616
   <212> DNA
   <213> Homo sapiens
<400> 6
   ccccggcgca gcgcggccgc agcagcctcc gccccccgca cggtgtgagc gcccgacgcg      60
   gccgaggcgg ccggagtccc gagctagccc cggcggccgc cgccgcccag accggacgac 120
   aggccacctc gtcggcgtcc gcccgagtcc ccgcctcgcc gccaacgcca caaccaccgc 180
   gcacggcccc ctgactccgt ccagtattga tcgggagagc cggagcgagc tcttcgggga 240
   gcagcgatgc gaccctccgg gacggccggg gcagcgctcc tggcgctgct ggctgcgctc 300
   tgcccggcga gtcgggctct ggaggaaaag aaagtttgcc aaggcacgag taacaagctc 360
   acgcagttgg gcacttttga agatcatttt ctcagcctcc agaggatgtt caataactgt 420
   gaggtggtcc ttgggaattt ggaaattacc tatgtgcaga ggaattatga tctttccttc 480
   ttaaagacca tccaggaggt ggctggttat gtcctcattg ccctcaacac agtggagcga 540
   attcctttgg aaaacctgca gatcatcaga ggaaatatgt actacgaaaa ttcctatgcc 600
   ttagcagtct tatctaacta tgatgcaaat aaaaccggac tgaaggagct gcccatgaga 660
   aatttacagg aaatcctgca tggcgccgtg cggttcagca acaaccctgc cctgtgcaac 720
   gtggagagca tccagtggcg ggacatagtc agcagtgact ttctcagcaa catgtcgatg 780
   gacttccaga accacctggg cagctgccaa aagtgtgatc caagctgtcc caatgggagc 840
   tgctggggtg caggagagga gaactgccag aaactgacca aaatcatctg tgcccagcag 900
   tgctccgggc gctgccgtgg caagtccccc agtgactgct gccacaacca gtgtgctgca 960
   ggctgcacag gcccccggga gagcgactgc ctggtctgcc gcaaattccg agacgaagcc 1020
   acgtgcaagg acacctgccc cccactcatg ctctacaacc ccaccacgta ccagatggat 1080
   gtgaaccccg agggcaaata cagctttggt gccacctgcg tgaagaagtg tccccgtaat 1140
   tatgtggtga cagatcacgg ctcgtgcgtc cgagcctgtg gggccgacag ctatgagatg 1200
   gaggaagacg gcgtccgcaa gtgtaagaag tgcgaagggc cttgccgcaa agtgtgtaac 1260
   ggaataggta ttggtgaatt taaagactca ctctccataa atgctacgaa tattaaacac 1320
   ttcaaaaact gcacctccat cagtggcgat ctccacatcc tgccggtggc atttaggggt 1380
   gactccttca cacatactcc tcctctggat ccacaggaac tggatattct gaaaaccgta 1440
   aaggaaatca cagggttttt gctgattcag gcttggcctg aaaacaggac ggacctccat 1500
   gcctttgaga acctagaaat catacgcggc aggaccaagc aacatggtca gttttctctt 1560
   gcagtcgtca gcctgaacat aacatccttg ggattacgct ccctcaagga gataagtgat 1620
   ggagatgtga taatttcagg aaacaaaaat ttgtgctatg caaatacaat aaactggaaa 1680
   aaactgtttg ggacctccgg tcagaaaacc aaaattataa gcaacagagg tgaaaacagc 1740
   tgcaaggcca caggccaggt ctgccatgcc ttgtgctccc ccgagggctg ctggggcccg 1800
   gagcccaggg actgcgtctc ttgccggaat gtcagccgag gcagggaatg cgtggacaag 1860
   tgcaaccttc tggagggtga gccaagggag tttgtggaga actctgagtg catacagtgc 1920
   cacccagagt gcctgcctca ggccatgaac atcacctgca caggacgggg accagacaac 1980
   tgtatccagt gtgcccacta cattgacggc ccccactgcg tcaagacctg cccggcagga 2040
   gtcatgggag aaaacaacac cctggtctgg aagtacgcag acgccggcca tgtgtgccac 2100
   ctgtgccatc caaactgcac ctacggatgc actgggccag gtcttgaagg ctgtccaacg 2160
   aatgggccta agatcccgtc catcgccact gggatggtgg gggccctcct cttgctgctg 2220
   gtggtggccc tggggatcgg cctcttcatg cgaaggcgcc acatcgttcg gaagcgcacg 2280
   ctgcggaggc tgctgcagga gagggagctt gtggagcctc ttacacccag tggagaagct 2340
   cccaaccaag ctctcttgag gatcttgaag gaaactgaat tcaaaaagat caaagtgctg 2400
   ggctccggtg cgttcggcac ggtgtataag ggactctgga tcccagaagg tgagaaagtt 2460
   aaaattcccg tcgctatcaa ggaattaaga gaagcaacat ctccgaaagc caacaaggaa 2520
   atcctcgatg aagcctacgt gatggccagc gtggacaacc cccacgtgtg ccgcctgctg 2580
   ggcatctgcc tcacctccac cgtgcagctc atcacgcagc tcatgccctt cggctgcctc 2640
   ctggactatg tccgggaaca caaagacaat attggctccc agtacctgct caactggtgt 2700
   gtgcagatcg caaagggcat gaactacttg gaggaccgtc gcttggtgca ccgcgacctg 2760
   gcagccagga acgtactggt gaaaacaccg cagcatgtca agatcacaga ttttgggctg 2820
   gccaaactgc tgggtgcgga agagaaagaa taccatgcag aaggaggcaa agtgcctatc 2880
   aagtggatgg cattggaatc aattttacac agaatctata cccaccagag tgatgtctgg 2940
   agctacgggg tgaccgtttg ggagttgatg acctttggat ccaagccata tgacggaatc 3000
   cctgccagcg agatctcctc catcctggag aaaggagaac gcctccctca gccacccata 3060
   tgtaccatcg atgtctacat gatcatggtc aagtgctgga tgatagacgc agatagtcgc 3120
   ccaaagttcc gtgagttgat catcgaattc tccaaaatgg cccgagaccc ccagcgctac 3180
   cttgtcattc agggggatga aagaatgcat ttgccaagtc ctacagactc caacttctac 3240
   cgtgccctga tggatgaaga agacatggac gacgtggtgg atgccgacga gtacctcatc 3300
   ccacagcagg gcttcttcag cagcccctcc acgtcacgga ctcccctcct gagctctctg 3360
   agtgcaacca gcaacaattc caccgtggct tgcattgata gaaatgggct gcaaagctgt 3420
   cccatcaagg aagacagctt cttgcagcga tacagctcag accccacagg cgccttgact 3480
   gaggacagca tagacgacac cttcctccca gtgcctgaat acataaacca gtccgttccc 3540
   aaaaggcccg ctggctctgt gcagaatcct gtctatcaca atcagcctct gaaccccgcg 3600
   cccagcagag acccacacta ccaggacccc cacagcactg cagtgggcaa ccccgagtat 3660
   ctcaacactg tccagcccac ctgtgtcaac agcacattcg acagccctgc ccactgggcc 3720
   cagaaaggca gccaccaaat tagcctggac aaccctgact accagcagga cttctttccc 3780
   aaggaagcca agccaaatgg catctttaag ggctccacag ctgaaaatgc agaataccta 3840
   agggtcgcgc cacaaagcag tgaatttatt ggagcatgac cacggaggat agtatgagcc 3900
   ctaaaaatcc agactctttc gatacccagg accaagccac agcaggtcct ccatcccaac 3960
   agccatgccc gcattagctc ttagacccac agactggttt tgcaacgttt acaccgacta 4020
   gccaggaagt acttccacct cgggcacatt ttgggaagtt gcattccttt gtcttcaaac 4080
   tgtgaagcat ttacagaaac gcatccagca agaatattgt ccctttgagc agaaatttat 4140
   ctttcaaaga ggtatatttg aaaaaaaaaa aaagtatatg tgaggatttt tattgattgg 4200
   ggatcttgga gtttttcatt gtcgctattg atttttactt caatgggctc ttccaacaag 4260
   gaagaagctt gctggtagca cttgctaccc tgagttcatc caggcccaac tgtgagcaag 4320
   gagcacaagc cacaagtctt ccagaggatg cttgattcca gtggttctgc ttcaaggctt 4380
   ccactgcaaa acactaaaga tccaagaagg ccttcatggc cccagcaggc cggatcggta 4440
   ctgtatcaag tcatggcagg tacagtagga taagccactc tgtcccttcc tgggcaaaga 4500
   agaaacggag gggatggaat tcttccttag acttactttt gtaaaaatgt ccccacggta 4560
   cttactcccc actgatggac cagtggtttc cagtcatgag cgttagactg acttgtttgt 4620
   cttccattcc attgttttga aactcagtat gctgcccctg tcttgctgtc atgaaatcag 4680
   caagagagga tgacacatca aataataact cggattccag cccacattgg attcatcagc 4740
   atttggacca atagcccaca gctgagaatg tggaatacct aaggatagca ccgcttttgt 4800
   tctcgcaaaa acgtatctcc taatttgagg ctcagatgaa atgcatcagg tcctttgggg 4860
   catagatcag aagactacaa aaatgaagct gctctgaaat ctcctttagc catcacccca 4920
   accccccaaa attagtttgt gttacttatg gaagatagtt ttctcctttt acttcacttc 4980
   aaaagctttt tactcaaaga gtatatgttc cctccaggtc agctgccccc aaaccccctc 5040
   cttacgcttt gtcacacaaa aagtgtctct gccttgagtc atctattcaa gcacttacag 5100
   ctctggccac aacagggcat tttacaggtg cgaatgacag tagcattatg agtagtgtgg 5160
   aattcaggta gtaaatatga aactagggtt tgaaattgat aatgctttca caacatttgc 5220
   agatgtttta gaaggaaaaa agttccttcc taaaataatt tctctacaat tggaagattg 5280
   gaagattcag ctagttagga gcccaccttt tttcctaatc tgtgtgtgcc ctgtaacctg 5340
   actggttaac agcagtcctt tgtaaacagt gttttaaact ctcctagtca atatccaccc 5400
   catccaattt atcaaggaag aaatggttca gaaaatattt tcagcctaca gttatgttca 5460
   gtcacacaca catacaaaat gttccttttg cttttaaagt aatttttgac tcccagatca 5520
   gtcagagccc ctacagcatt gttaagaaag tatttgattt ttgtctcaat gaaaataaaa 5580
   ctatattcat ttccactcta aaaaaaaaaa aaaaaa 5616
<210> 7
   <211> 2239
   <212> DNA
   <213> Homo sapiens
<400> 7
   ccccggcgca gcgcggccgc agcagcctcc gccccccgca cggtgtgagc gcccgacgcg 60
   gccgaggcgg ccggagtccc gagctagccc cggcggccgc cgccgcccag accggacgac 120
   aggccacctc gtcggcgtcc gcccgagtcc ccgcctcgcc gccaacgcca caaccaccgc 180
   gcacggcccc ctgactccgt ccagtattga tcgggagagc cggagcgagc tcttcgggga 240
   gcagcgatgc gaccctccgg gacggccggg gcagcgctcc tggcgctgct ggctgcgctc 300
   tgcccggcga gtcgggctct ggaggaaaag aaagtttgcc aaggcacgag taacaagctc 360
   acgcagttgg gcacttttga agatcatttt ctcagcctcc agaggatgtt caataactgt 420
   gaggtggtcc ttgggaattt ggaaattacc tatgtgcaga ggaattatga tctttccttc 480
   ttaaagacca tccaggaggt ggctggttat gtcctcattg ccctcaacac agtggagcga 540
   attcctttgg aaaacctgca gatcatcaga ggaaatatgt actacgaaaa ttcctatgcc 600
   ttagcagtct tatctaacta tgatgcaaat aaaaccggac tgaaggagct gcccatgaga 660
   aatttacagg aaatcctgca tggcgccgtg cggttcagca acaaccctgc cctgtgcaac 720
   gtggagagca tccagtggcg ggacatagtc agcagtgact ttctcagcaa catgtcgatg 780
   gacttccaga accacctggg cagctgccaa aagtgtgatc caagctgtcc caatgggagc 840
   tgctggggtg caggagagga gaactgccag aaactgacca aaatcatctg tgcccagcag 900
   tgctccgggc gctgccgtgg caagtccccc agtgactgct gccacaacca gtgtgctgca 960
   ggctgcacag gcccccggga gagcgactgc ctggtctgcc gcaaattccg agacgaagcc 1020
   acgtgcaagg acacctgccc cccactcatg ctctacaacc ccaccacgta ccagatggat 1080
   gtgaaccccg agggcaaata cagctttggt gccacctgcg tgaagaagtg tccccgtaat 1140
   tatgtggtga cagatcacgg ctcgtgcgtc cgagcctgtg gggccgacag ctatgagatg 1200
   gaggaagacg gcgtccgcaa gtgtaagaag tgcgaagggc cttgccgcaa agtgtgtaac 1260
   ggaataggta ttggtgaatt taaagactca ctctccataa atgctacgaa tattaaacac 1320
   ttcaaaaact gcacctccat cagtggcgat ctccacatcc tgccggtggc atttaggggt 1380
   gactccttca cacatactcc tcctctggat ccacaggaac tggatattct gaaaaccgta 1440
   aaggaaatca cagggttttt gctgattcag gcttggcctg aaaacaggac ggacctccat 1500
   gcctttgaga acctagaaat catacgcggc aggaccaagc aacatggtca gttttctctt 1560
   gcagtcgtca gcctgaacat aacatccttg ggattacgct ccctcaagga gataagtgat 1620
   ggagatgtga taatttcagg aaacaaaaat ttgtgctatg caaatacaat aaactggaaa 1680
   aaactgtttg ggacctccgg tcagaaaacc aaaattataa gcaacagagg tgaaaacagc 1740
   tgcaaggcca caggccaggt ctgccatgcc ttgtgctccc ccgagggctg ctggggcccg 1800
   gagcccaggg actgcgtctc ttgccggaat gtcagccgag gcagggaatg cgtggacaag 1860
   tgcaaccttc tggagggtga gccaagggag tttgtggaga actctgagtg catacagtgc 1920
   cacccagagt gcctgcctca ggccatgaac atcacctgca caggacgggg accagacaac 1980
   tgtatccagt gtgcccacta cattgacggc ccccactgcg tcaagacctg cccggcagga 2040
   gtcatgggag aaaacaacac cctggtctgg aagtacgcag acgccggcca tgtgtgccac    2100
   ctgtgccatc caaactgcac ctacgggtcc taataaatct tcactgtctg actttagtct    2160
   cccactaaaa ctgcatttcc tttctacaat ttcaatttct ccctttgctt caaataaagt    2220
   cctgacacta ttcatttga                                                 2239
<210> 8
   <211> 1595
   <212> DNA
   <213> Homo sapiens
<400> 8
   ccccggcgca gcgcggccgc agcagcctcc gccccccgca cggtgtgagc gcccgacgcg      60
   gccgaggcgg ccggagtccc gagctagccc cggcggccgc cgccgcccag accggacgac      120
   aggccacctc gtcggcgtcc gcccgagtcc ccgcctcgcc gccaacgcca caaccaccgc      180
   gcacggcccc ctgactccgt ccagtattga tcgggagagc cggagcgagc tcttcgggga      240
   gcagcgatgc gaccctccgg gacggccggg gcagcgctcc tggcgctgct ggctgcgctc      300
   tgcccggcga gtcgggctct ggaggaaaag aaagtttgcc aaggcacgag taacaagctc      360
   acgcagttgg gcacttttga agatcatttt ctcagcctcc agaggatgtt caataactgt      420
   gaggtggtcc ttgggaattt ggaaattacc tatgtgcaga ggaattatga tctttccttc      480
   ttaaagacca tccaggaggt ggctggttat gtcctcattg ccctcaacac agtggagcga      540
   attcctttgg aaaacctgca gatcatcaga ggaaatatgt actacgaaaa ttcctatgcc      600
   ttagcagtct tatctaacta tgatgcaaat aaaaccggac tgaaggagct gcccatgaga      660
   aatttacagg aaatcctgca tggcgccgtg cggttcagca acaaccctgc cctgtgcaac      720
   gtggagagca tccagtggcg ggacatagtc agcagtgact ttctcagcaa catgtcgatg      780
   gacttccaga accacctggg cagctgccaa aagtgtgatc caagctgtcc caatgggagc      840
   tgctggggtg caggagagga gaactgccag aaactgacca aaatcatctg tgcccagcag      900
   tgctccgggc gctgccgtgg caagtccccc agtgactgct gccacaacca gtgtgctgca      960
   ggctgcacag gcccccggga gagcgactgc ctggtctgcc gcaaattccg agacgaagcc     1020
   acgtgcaagg acacctgccc cccactcatg ctctacaacc ccaccacgta ccagatggat     1080
   gtgaaccccg agggcaaata cagctttggt gccacctgcg tgaagaagtg tccccgtaat     1140
   tatgtggtga cagatcacgg ctcgtgcgtc cgagcctgtg gggccgacag ctatgagatg     1200
   gaggaagacg gcgtccgcaa gtgtaagaag tgcgaagggc cttgccgcaa agtgtgtaac     1260
   ggaataggta ttggtgaatt taaagactca ctctccataa atgctacgaa tattaaacac     1320
   ttcaaaaact gcacctccat cagtggcgat ctccacatcc tgccggtggc atttaggggt     1380
   gactccttca cacatactcc tcctctggat ccacaggaac tggatattct gaaaaccgta     1440
   aaggaaatca caggtttgag ctgaattatc acatgaatat aaatgggaaa tcagtgtttt     1500
   agagagagaa cttttcgaca tatttcctgt tcccttggaa taaaaacatt tcttctgaaa     1560
   ttttaccgtt aaaaaaaaaa aaaaaaaaaa aaaaa                                1595
<210> 9
   <211> 2865
   <212> DNA
   <213> Homo sapiens
<400> 9
   ccccggcgca gcgcggccgc agcagcctcc gccccccgca cggtgtgagc gcccgacgcg 60
   gccgaggcgg ccggagtccc gagctagccc cggcggccgc cgccgcccag accggacgac 120
   aggccacctc gtcggcgtcc gcccgagtcc ccgcctcgcc gccaacgcca caaccaccgc 180
   gcacggcccc ctgactccgt ccagtattga tcgggagagc cggagcgagc tcttcgggga 240
   gcagcgatgc gaccctccgg gacggccggg gcagcgctcc tggcgctgct ggctgcgctc 300
   tgcccggcga gtcgggctct ggaggaaaag aaagtttgcc aaggcacgag taacaagctc 360
   acgcagttgg gcacttttga agatcatttt ctcagcctcc agaggatgtt caataactgt 420
   gaggtggtcc ttgggaattt ggaaattacc tatgtgcaga ggaattatga tctttccttc 480
   ttaaagacca tccaggaggt ggctggttat gtcctcattg ccctcaacac agtggagcga 540
   attcctttgg aaaacctgca gatcatcaga ggaaatatgt actacgaaaa ttcctatgcc 600
   ttagcagtct tatctaacta tgatgcaaat aaaaccggac tgaaggagct gcccatgaga 660
   aatttacagg aaatcctgca tggcgccgtg cggttcagca acaaccctgc cctgtgcaac 720
   gtggagagca tccagtggcg ggacatagtc agcagtgact ttctcagcaa catgtcgatg 780
   gacttccaga accacctggg cagctgccaa aagtgtgatc caagctgtcc caatgggagc 840
   tgctggggtg caggagagga gaactgccag aaactgacca aaatcatctg tgcccagcag 900
   tgctccgggc gctgccgtgg caagtccccc agtgactgct gccacaacca gtgtgctgca 960
   ggctgcacag gcccccggga gagcgactgc ctggtctgcc gcaaattccg agacgaagcc 1020
   acgtgcaagg acacctgccc cccactcatg ctctacaacc ccaccacgta ccagatggat 1080
   gtgaaccccg agggcaaata cagctttggt gccacctgcg tgaagaagtg tccccgtaat 1140
   tatgtggtga cagatcacgg ctcgtgcgtc cgagcctgtg gggccgacag ctatgagatg 1200
   gaggaagacg gcgtccgcaa gtgtaagaag tgcgaagggc cttgccgcaa agtgtgtaac 1260
   ggaataggta ttggtgaatt taaagactca ctctccataa atgctacgaa tattaaacac 1320
   ttcaaaaact gcacctccat cagtggcgat ctccacatcc tgccggtggc atttaggggt 1380
   gactccttca cacatactcc tcctctggat ccacaggaac tggatattct gaaaaccgta 1440
   aaggaaatca cagggttttt gctgattcag gcttggcctg aaaacaggac ggacctccat 1500
   gcctttgaga acctagaaat catacgcggc aggaccaagc aacatggtca gttttctctt 1560
   gcagtcgtca gcctgaacat aacatccttg ggattacgct ccctcaagga gataagtgat 1620
   ggagatgtga taatttcagg aaacaaaaat ttgtgctatg caaatacaat aaactggaaa 1680
   aaactgtttg ggacctccgg tcagaaaacc aaaattataa gcaacagagg tgaaaacagc 1740
   tgcaaggcca caggccaggt ctgccatgcc ttgtgctccc ccgagggctg ctggggcccg 1800
   gagcccaggg actgcgtctc ttgccggaat gtcagccgag gcagggaatg cgtggacaag 1860
   tgcaaccttc tggagggtga gccaagggag tttgtggaga actctgagtg catacagtgc 1920
   cacccagagt gcctgcctca ggccatgaac atcacctgca caggacgggg accagacaac 1980
   tgtatccagt gtgcccacta cattgacggc ccccactgcg tcaagacctg cccggcagga 2040
   gtcatgggag aaaacaacac cctggtctgg aagtacgcag acgccggcca tgtgtgccac 2100
   ctgtgccatc caaactgcac ctacgggcca ggaaatgaga gtctcaaagc catgttattc 2160
   tgccttttta aactatcatc ctgtaatcaa agtaatgatg gcagcgtgtc ccaccagagc 2220
   gggagcccag ctgctcagga gtcatgctta ggatggatcc cttctcttct gccgtcagag 2280
   tttcagctgg gttggggtgg atgcagccac ctccatgcct ggccttctgc atctgtgatc 2340
   atcacggcct cctcctgcca ctgagcctca tgccttcacg tgtctgttcc ccccgctttt 2400
   cctttctgcc acccctgcac gtgggccgcc aggttcccaa gagtatccta cccatttcct 2460
   tccttccact ccctttgcca gtgcctctca ccccaactag tagctaacca tcacccccag 2520
   gactgacctc ttcctcctcg ctgccagatg attgttcaaa gcacagaatt tgtcagaaac 2580
   ctgcagggac tccatgctgc cagccttctc cgtaattagc atggccccag tccatgcttc 2640
   tagccttggt tccttctgcc cctctgtttg aaattctaga gccagctgtg ggacaattat 2700
   ctgtgtcaaa agccagatgt gaaaacatct caataacaaa ctggctgctt tgttcaatgc 2760
   tagaacaacg cctgtcacag agtagaaact caaaaatatt tgctgagtga atgaacaaat 2820
   gaataaatgc ataataaata attaaccacc aatccaacat ccaga 2865
<210> 10
   <211> 5983
   <212> DNA
   <213> Mus musculus
<400> 10
   ctcccccagt cccgacccga gctaactaga cgtctgggca gccccagcgc aacgcgcagc      60
   agcctccctc ctcttcttcc cgcactgtgc gctcctcctg ggctagggcg tctggatcga     120
   gtcccggagg ctaccgcctc ccagacagac gacaggtcac ctggacgcga gcctgtgtcc     180
   gggtctcgtc gttgccggcg cagtcactgg gcacaaccgt gggactccgt ctgtctcgga     240
   ttaatcccgg agagccagag ccaacctctc ccggtcagag atgcgaccct cagggaccgc     300
   gagaaccaca ctgctggtgt tgctgaccgc gctctgcgcc gcaggtgggg cgttggagga     360
   aaagaaagtc tgccaaggca caagtaacag gctcacccaa ctgggcactt ttgaagacca     420
   ctttctgagc ctgcagagga tgtacaacaa ctgtgaagtg gtccttggga acttggaaat     480
   tacctatgtg caaaggaatt acgacctttc cttcttaaag accatccagg aggtggccgg 540
   ctatgtcctc attgccctca acaccgtgga gagaatccct ttggagaacc tgcagatcat 600
   caggggaaat gctctttatg aaaacaccta tgccttagcc atcctgtcca actatgggac 660
   aaacagaact gggcttaggg aactgcccat gcggaactta caggaaatcc tgattggtgc 720
   tgtgcgattc agcaacaacc ccatcctctg caatatggat actatccagt ggagggacat 780
   cgtccaaaac gtctttatga gcaacatgtc aatggactta cagagccatc cgagcagttg 840
   ccccaaatgt gatccaagct gtcccaatgg aagctgctgg ggaggaggag aggagaactg 900
   ccagaaattg accaaaatca tctgtgccca gcaatgttcc catcgctgtc gtggcaggtc 960
   ccccagtgac tgctgccaca accaatgtgc tgcggggtgt acagggcccc gagagagtga 1020
   ctgtctggtc tgccaaaagt tccaagatga ggccacatgc aaagacacct gcccaccact 1080
   catgctgtac aaccccacca cctatcagat ggatgtcaac cctgaaggga agtacagctt 1140
   tggtgccacc tgtgtgaaga agtgcccccg aaactacgtg gtgacagatc atggctcatg 1200
   tgtccgagcc tgtgggcctg actactacga agtggaagaa gatggcatcc gcaagtgtaa 1260
   aaaatgtgat gggccctgtc gcaaagtttg taatggcata ggcattggtg aatttaaaga 1320
   cacactctcc ataaatgcta caaacatcaa acacttcaaa tactgcactg ccatcagcgg 1380
   ggaccttcac atcctgccag tggcctttaa gggggattct ttcacgcgca ctcctcctct 1440
   agacccacga gaactagaaa ttctaaaaac cgtaaaggaa ataacaggct ttttgctgat 1500
   tcaggcttgg cctgataact ggactgacct ccatgctttc gagaacctag aaataatacg 1560
   tggcagaaca aagcaacatg gtcagttttc tttggcggtc gttggcctga acatcacatc 1620
   actggggctg cgttccctca aggagatcag tgatggggat gtgatcattt ctggaaaccg 1680
   aaatttgtgc tacgcaaaca caataaactg gaaaaaactc ttcgggacac ccaatcagaa 1740
   aaccaaaatc atgaacaaca gagctgagaa agactgcaag gccgtgaacc acgtctgcaa 1800
   tcctttatgc tcctcggaag gctgctgggg ccctgagccc agggactgtg tctcctgcca 1860
   gaatgtgagc agaggcaggg agtgcgtgga gaaatgcaac atcctggagg gggaaccaag 1920
   ggagtttgtg gaaaattctg aatgcatcca gtgccatcca gaatgtctgc cccaggccat 1980
   gaacatcacc tgtacaggca ggggaccaga caactgcatc cagtgtgccc actacattga 2040
   tggcccacac tgtgtcaaga cctgcccagc tggcatcatg ggagagaaca acactctggt 2100
   ctggaagtat gcagatgcca ataatgtctg ccacctatgc cacgccaact gtacctatgg 2160
   atgtgctggg ccaggtcttc aaggatgtga agtgtggcca tctgggccaa agataccatc 2220
   tattgccact gggattgtgg gtggcctcct cttcatagtg gtggtggccc ttgggattgg 2280
   cctattcatg cgaagacgtc acattgttcg aaagcgtaca ctacgccgcc tgcttcaaga 2340
   gagagagctc gtggaacctc tcacacccag cggagaagct ccaaaccaag cccacttgag 2400
   gatattaaag gaaacagaat tcaaaaagat caaagttctg ggttcgggag catttggcac     2460
   agtgtataag ggtctctgga tcccagaagg tgagaaagta aaaatcccgg tggccatcaa     2520
   ggagttaaga gaagccacat ctccaaaagc caacaaagaa atccttgacg aagcctatgt     2580
   gatggctagt gtggacaacc ctcatgtatg ccgcctcctg ggcatctgtc tgacctccac     2640
   tgtccagctc attacacagc tcatgcccta cggttgcctc ctggactacg tccgagaaca     2700
   caaggacaac attggctccc agtacctcct caactggtgt gtgcagattg caaagggcat     2760
   gaactacctg gaagatcggc gtttggtgca ccgtgacttg gcagccagga atgtactggt     2820
   gaagacacca cagcatgtca agatcacaga ttttgggctg gccaaactgc ttggtgctga     2880
   agagaaagaa tatcatgccg aggggggcaa agtgcctatc aagtggatgg ctttggaatc     2940
   aattttacac cgaatttata cacaccaaag tgatgtctgg agctatggtg tcactgtgtg     3000
   ggaactgatg acctttgggt ccaagcctta tgatggaatc ccagcaagtg acatctcatc     3060
   catcctagag aaaggagagc gccttccaca gccacctatc tgcaccatcg atgtctacat     3120
   gatcatggtc aagtgctgga tgatagatgc tgatagccgc ccaaagttcc gagagttgat     3180
   tcttgaattc tccaaaatgg cccgagaccc acagcgctac cttgttatcc agggggatga     3240
   aagaatgcat ttgccaagcc ctacagactc caacttttac cgagccctga tggatgaaga     3300
   ggacatggag gatgtagttg atgctgatga gtatcttatc ccacagcaag gcttcttcaa     3360
   cagcccgtcc acgtcgagga ctcccctctt gagttctctg agtgcaacta gcaacaattc     3420
   cactgtggct tgcattaata gaaatgggag ctgccgtgtc aaagaagacg ccttcttgca     3480
   gcggtacagc tccgacccca caggtgctgt aacagaggac aacatagatg acgcattcct     3540
   ccctgtacct gaatatgtaa accaatctgt tcccaagagg ccagcaggct ctgtgcagaa     3600
   ccctgtctat cacaatcagc ccctgcatcc agctcctgga agagacctgc attatcaaaa     3660
   tccccacagc aatgcagtgg gcaaccctga gtatctcaac actgcccagc ctacctgtct     3720
   cagtagtggg tttaacagcc ctgcactctg gatccagaaa ggcagtcacc aaatgagcct     3780
   agacaaccct gactaccagc aggacttctt ccccaaggaa accaagccaa atggcatatt     3840
   taagggcccc acagctgaaa atgcagagta cctacgggtg gcacctccaa gcagtgagtt     3900
   tattggagca tgacaagaag gggcatcata ccagctataa aatgtctgga ctttctagaa     3960
   tcccaggacc aactatggca gcacctccac ttctggtagc catgcccacg ctgtgtcaaa     4020
   tgtcactcag actggcttta aagcataact ctgatgggct ttgtcactga gccaagaagt     4080
   gggcctctct cctgatgcac tttgggaagt tgaaggtaca tcaattgatc ttcgaactgt     4140
   gaagattcca caaaaaaggt atccatcgag aacattgtcc attggaacag aagtttgcct     4200
   catggtgagg tacatatggg aaaaaaacag acatatggag cttatattta gggaactttg     4260
   ggattcttgt ctttattgat ttgattgatg cactcttgta gtctggtaca cagagttgcc     4320
   tggagccaac tgaccagaca gttggttcca ccagctctgc atcaagacac ttccgtggca     4380
   agacaactaa atgtataaga agtccatgga tgccctgagc aggccacact tgtacagcat 4440
   taaaccatgg cagatacaat aggataagcc actttgttac ttactggggc tgggagaaga 4500
   ggaatgacgg ggtagaattt tccctcagac gtacttttta tataaatatg tccctggcac 4560
   ctaacacgcg ctagtttacc agtgttttct attagacttc cttctatgtt ttctgtttca 4620
   ttgttttgag ttgtaaatat gtgttcctgt cttcatttca tgaagtaaac aaacaaacaa 4680
   aaaacccagt attaagtatt atcaaagaac aaccatgatt ccacattcga acccattcaa 4740
   accatcagta ttgtgaccaa aagcctttaa ctaagaagga gtaaccatgc aaaaatccat 4800
   agaggaattt aacccaaaat tttagtctca gcattgtgtc tgctgaggtg tgtatatgag 4860
   actacgaaag tgaactactc ttcaaatcca ctttgccttc actcctctat accctaaatc 4920
   tagtgtaaac cacacatgga ggataacttt tttttttaat tttaaaagtg tttattagat 4980
   atgtttttct tcctggtaaa ctgcagccaa acatcagtta agagccattt ttgataaaca 5040
   ctatcacaat gatctcggga tccatccttt ccgatttacc aagtgatgga tagacgtgaa 5100
   ctcataaaca ctacccataa gacaaaacaa tgagtgccag acaagacatc agccaggcac 5160
   cagagcacag agcaggactg ggcaatctgt tggagatatc tagaaagttc acaaaggaaa 5220
   caagattgtc cactaccttg tgagatctag cagtcataaa taccagggaa atggaaagtg 5280
   tgtttcctta cagcaccagg tcttcgatct tcctaatgct gtgacccttt aatacagttt 5340
   gccatgttgt ggtgaccccc aaccataaaa ttatttttgt tgctacttca taactgtaaa 5400
   tttgctactc ttacagacca caatgtaaat atctgatatg ctatctgata tgcaggctat 5460
   ctgacagagg tcgcaacccg caggttgaga gccactgcct tcaaggcttt aatcaagaga 5520
   gtagtgagct gagggcttta ctggtaagtc aggggcaagt ccaactcaat catcctcaca 5580
   tactggctgc tccctcaggc ctgagaatga ggcttgcagc atcctctggt ttcctaaccg 5640
   ttatccatcc ctgactctca tctctgaaaa tagatgtcat ccatgaaatt aaggagtgag 5700
   aatattaagc agcatttata gagctcaaaa ttccatgtca tcaccaggaa gtgccatgtt 5760
   gatcacagag aacacagagg agacatatag acagggtttt gctcaaaatt gggatataga 5820
   atgagcctgt caggtaccta tcaggagcgg taatccgtga gagagaaccg ttgcaagcca 5880
   ctctaactgt agcaatgaaa ccctagtatt tttgtacttt gaaatacttt cttataacaa 5940
   aataaagtag caaaaaaact gttcaaaaaa aaaaaaaaaa aaa 5983
<210> 11
   <211> 2678
   <212> DNA
   <213> Mus musculus
<400> 11
   ctcccccagt cccgacccga gctaactaga cgtctgggca gccccagcgc aacgcgcagc        60
   agcctccctc ctcttcttcc cgcactgtgc gctcctcctg ggctagggcg tctggatcga       120
   gtcccggagg ctaccgcctc ccagacagac gacaggtcac ctggacgcga gcctgtgtcc 180
   gggtctcgtc gttgccggcg cagtcactgg gcacaaccgt gggactccgt ctgtctcgga 240
   ttaatcccgg agagccagag ccaacctctc ccggtcagag atgcgaccct cagggaccgc 300
   gagaaccaca ctgctggtgt tgctgaccgc gctctgcgcc gcaggtgggg cgttggagga 360
   aaagaaagtc tgccaaggca caagtaacag gctcacccaa ctgggcactt ttgaagacca 420
   ctttctgagc ctgcagagga tgtacaacaa ctgtgaagtg gtccttggga acttggaaat 480
   tacctatgtg caaaggaatt acgacctttc cttcttaaag accatccagg aggtggccgg 540
   ctatgtcctc attgccctca acaccgtgga gagaatccct ttggagaacc tgcagatcat 600
   caggggaaat gctctttatg aaaacaccta tgccttagcc atcctgtcca actatgggac 660
   aaacagaact gggcttaggg aactgcccat gcggaactta caggaaatcc tgattggtgc 720
   tgtgcgattc agcaacaacc ccatcctctg caatatggat actatccagt ggagggacat 780
   cgtccaaaac gtctttatga gcaacatgtc aatggactta cagagccatc cgagcagttg 840
   ccccaaatgt gatccaagct gtcccaatgg aagctgctgg ggaggaggag aggagaactg 900
   ccagaaattg accaaaatca tctgtgccca gcaatgttcc catcgctgtc gtggcaggtc 960
   ccccagtgac tgctgccaca accaatgtgc tgcggggtgt acagggcccc gagagagtga 1020
   ctgtctggtc tgccaaaagt tccaagatga ggccacatgc aaagacacct gcccaccact 1080
   catgctgtac aaccccacca cctatcagat ggatgtcaac cctgaaggga agtacagctt 1140
   tggtgccacc tgtgtgaaga agtgcccccg aaactacgtg gtgacagatc atggctcatg 1200
   tgtccgagcc tgtgggcctg actactacga agtggaagaa gatggcatcc gcaagtgtaa 1260
   aaaatgtgat gggccctgtc gcaaagtttg taatggcata ggcattggtg aatttaaaga 1320
   cacactctcc ataaatgcta caaacatcaa acacttcaaa tactgcactg ccatcagcgg 1380
   ggaccttcac atcctgccag tggcctttaa gggggattct ttcacgcgca ctcctcctct 1440
   agacccacga gaactagaaa ttctaaaaac cgtaaaggaa ataacaggct ttttgctgat 1500
   tcaggcttgg cctgataact ggactgacct ccatgctttc gagaacctag aaataatacg 1560
   tggcagaaca aagcaacatg gtcagttttc tttggcggtc gttggcctga acatcacatc 1620
   actggggctg cgttccctca aggagatcag tgatggggat gtgatcattt ctggaaaccg 1680
   aaatttgtgc tacgcaaaca caataaactg gaaaaaactc ttcgggacac ccaatcagaa 1740
   aaccaaaatc atgaacaaca gagctgagaa agactgcaag gccgtgaacc acgtctgcaa 1800
   tcctttatgc tcctcggaag gctgctgggg ccctgagccc agggactgtg tctcctgcca 1860
   gaatgtgagc agaggcaggg agtgcgtgga gaaatgcaac atcctggagg gggaaccaag 1920
   ggagtttgtg gaaaattctg aatgcatcca gtgccatcca gaatgtctgc cccaggccat 1980
   gaacatcacc tgtacaggca ggggaccaga caactgcatc cagtgtgccc actacattga 2040
   tggcccacac tgtgtcaaga cctgcccagc tggcatcatg ggagagaaca acactctggt 2100
   ctggaagtat gcagatgcca ataatgtctg ccacctatgc cacgccaact gtacctatgg 2160
   atgtgctggg ccaggtcttc aaggatgtga agtgtggcca tctgggtacg ttcaatggca 2220
   gtggatctta aagacctttt ggatctaaga ccagaagcca tctctgactc ccctctcacc 2280
   ttccagtttc ttccaaatcc tctgggccag ccagaggtct cagattctgc cctcttgccc 2340
   tgtgcccacc ttgttgacca ctggacagca tatgtgatgg ctactgctag tgccagcttc 2400
   acaagaggtt aacactacgg actagccatt cttcctatgt atctgtttct gcaaatacag 2460
   ccgctttact taagtctcag cacttcttag tctcctcttt tcctctcagt agcccaaggg 2520
   gtcatgtcac aaacatggtg tgaagggcta ctttgtcaaa tgaaaaggtc tatcttgggg 2580
   ggcatttttt tcttttcttt ttttcttgaa acacattgcc cagcaaagcc aataaatttc 2640
   tctcatcatt ttgtttctga taaattctta ctattgat 2678
<210> 12
   <211> 4161
   <212> DNA
   <213> Rattus norvegicus
<400> 12
   ggaccgccac caagacaggc gacgggtcac ctggacgcga gtctgagtcc gggtcccgtc      60
   gtcgttgccg acgcagtcac cgggcacgac cgtgggactc agtctgactc ggattaatcc     120
   cggagagcca gagccaacga ctgccgagcc gggatgcgac cctcagggac tgcgagaacc     180
   aagctactgc tgctgctggc tgcgctctgc gccgcaggtg gggcgctgga ggaaaagaaa     240
   gtttgccaag gcacaagtaa caggctcacc caactaggca cctttgaaga ccactttctg     300
   agcctccaga ggatgttcaa caactgtgaa gtggtccttg gaaacttgga aatcacctat     360
   gtgcaaagga attatgacct ttccttctta aagaccatcc aggaggtggc tggctatgtt     420
   ctcattgccc tgaacaccgt ggagagaatc cctttggaga acctgcagat catcagggga     480
   aatgctctct acgaaaacac ctacgcctta gccgtcctgt ccaactatgg aaccaacaaa     540
   actgggctta gggaactgcc catgcggaac ttacaggaaa ttctgatcgg tgctgtgcga     600
   tttagcaaca accccatcct ctgcaatatg gagaccatcc agtggaggga catcgtccaa     660
   gatgtctttc tgagcaacat gtcaatggac gtacagcgcc acctgacggg ctgcccgaaa     720
   tgtgatccga gctgtcccaa tggaagctgc tggggaagag gagaggagaa ctgccagaaa     780
   ttgaccaaaa tcatctgcgc ccagcaatgt tcccggcgtt gtcgtggcag gtcccctagc     840
   gactgctgcc acaaccagtg tgccgcaggg tgtacagggc ccagagagag tgactgtctg     900
   gtctgccaca ggttccgaga tgaagccacg tgcaaagaca cctgcccacc actcatgctg     960
   tacaacccca ccacgtacca gatggatgtc aaccctgagg ggaagtacag ctttggtgcc    1020
   acctgtgtga agaaatgccc cagaaactac gtggtgacag atcacggctc gtgtgtccgg    1080
   gcctgtgggc cagactacta tgaagtagaa gaagatggag tcagcaagtg taaaaaatgt 1140
   gacgggccct gccgcaaagt ttgcaatggc ataggcattg gtgaatttaa agacacactc 1200
   tccataaatg ctacaaacat caaacacttc aagtactgca ctgccatcag tggggacctc 1260
   cacatcctgc cagtggcctt taagggggat tctttcaccc gcactcctcc tctagaccca 1320
   cgggaactag aaattctcaa aactgtgaag gaaataacag ggtttttgct gattcaggct 1380
   tggcctgaaa actggactga cctccatgct tttgagaacc tagaaataat tcgtggcaga 1440
   acaaagcaac atggtcagtt ttctctggcg gttgtcggcc tgaacataac atcgctgggg 1500
   ttgcgttccc tcaaggagat cagtgatggg gatgtgatta tttctgggaa ccgaaatttg 1560
   tgctacgcaa acactataaa ctggaaaaaa ctcttcggga cgcccaatca aaagaccaaa 1620
   atcatgaaca acagagctga aaaggactgc aaggccacga accacgtctg taatccttta 1680
   tgctcctcgg aaggctgctg gggccctgag cccacggact gtgtctcctg ccagaatgtg 1740
   agcagaggca gggagtgcgt ggacaagtgc aacatcctgg agggggaacc gagggagttt 1800
   gtggaaaatt ctgaatgcat ccagtgccat ccagaatgtc tgccccagac catgaacatc 1860
   acctgtacag gccgggggcc agacaactgc atcaagtgtg cccactatgt tgatggtccc 1920
   cactgtgtca agacctgccc ttcgggcatc atgggggaga acaacaccct ggtctggaag 1980
   tttgcagatg ccaataacgt ctgccacctc tgccatgcaa actgtaccta tggatgtgct 2040
   gggccaggcc ttaaaggatg tcaacaacca gaagggccaa agatcccatc catcgccact 2100
   gggattgtgg gtggcctcct cttcatagta gtggtggccc ttgggatcgg cctcttcatg 2160
   cgtcgacgtc agcttgtccg aaaacgtaca ctacgccgcc tgcttcaaga gagagagctc 2220
   gtggaacctc tcacacccag cggagaagct ccgaaccaag cccacttgag gatattaaag 2280
   gaaacagaat tcaaaaagat caaagttctg ggttcaggag catttggcac agtgtataag 2340
   ggtctctgga tcccagaagg cgagaaagtg aaaatccctg tggccatcaa ggagttaaga 2400
   gaagccacat ctcccaaagc caacaaggaa atccttgatg aagcctacgt gatggccagt 2460
   gtggacaacc ctcatgtatg ccgcctcctg ggcatctgtc tgacctccac tgtccagctc 2520
   attacacaac tcatgcccta tggttgcctc ctggactatg tccgagaaca taaggacaac 2580
   attggctccc agtacctact caactggtgt gtgcagattg caaagggcat gaactacctg 2640
   gaagaccggc gtttggtaca ccgtgacttg gcagccagga atgtactggt aaagacacca 2700
   cagcatgtca agatcacaga ttttggactg gccaaactgc ttggtgctga ggagaaagaa 2760
   taccatgcag aggggggcaa agtgcctatc aagtggatgg ctttggaatc aattttacac 2820
   cgaatttata cacaccaaag cgacgtctgg agctatggag tcaccgtgtg ggaactgatg 2880
   acctttgggt ccaagcctta tgatgggatc cctgcaagtg agatctcatc catcctagag 2940
   aaaggagagc gccttccaca gccacctatc tgcaccatcg acgtctacat gatcatggtc 3000
   aagtgctgga tgatagatgc tgatagccgc ccaaagttcc gagagttgat tctcgaattc 3060
   tccaaaatgg ccagagaccc acagcgctac cttgttatcc agggggatga aaggatgcat 3120
   ttgccgagcc ctacagactc caacttttac cgagccctga tggaggagga ggacatggaa 3180
   gacgtagttg atgctgatga atacctcatc ccacagcaag gcttcttcaa cagcccatcc 3240
   acgtcacgga ctccactctt gagctctctg agtgcaaata gcaacagttc cactgtggct 3300
   tgcattaata gaaatgggag ctgccgtgtc aaagaagacg ccttcttgca acggtatagc 3360
   tccgatccca ccagcgtcct gacagaggac aacatagatg acacattcct tcccgtgcct 3420
   gaatatataa accaatctgt tcccaagagg ccggctggct ctgtgcagaa cccagtctat 3480
   cacaatcagc ccctgcatcc agctcctgga agagacctgc attatcaaaa tccccatagc 3540
   aatgcggtga gcaaccctga gtatctcaac actgcccagc cgacctgcct cagtagtggg 3600
   tttgacagct ctgccctctg gatccagaaa ggcagccacc aaatgagcct ggacaaccct 3660
   gactaccagc aggacttctt tcccaaagaa gccaagccga atggcatctt taagggcccc 3720
   acagctgaaa atgcagagta cctgcgggtg gcaccgccaa gcagtgagtt tagtggagca 3780
   tgacattgaa gaggcattgt accagttaca aaccggactt tccagaggcc caggaccaag 3840
   ccatggcagc acctctgctc ctgacagcca tgtccacatt gtgtcaaatg tcaaaccctc 3900
   agactggctt taaagcataa ctctgacggg ctttgtcact gagccaagaa gtgggcccct 3960
   cccctgatgc tctttgggaa gttgaaggta tatctattgg tcttcgaact gtgaagattc 4020
   cactgaaagg tatccatcga gaacattgtc cttttggaac agaaggttgc ctcatggtga 4080
   ggtacatagg gggaaaaaaa acagacctat ggcgcttgct gacataggga actctgggat 4140
   tcttgtcttt attgatttgc t 4161
<210> 13
   <211> 4788
   <212> DNA
   <213> Canis familiaris
<400> 13
   atgctttcac cacccaagtg tgagcaagtg caaggccgta gaggcaccgc aggaccggga      60
   aaccgggaag agcagcggga tggggttgtc ctcctggggg gtgtcgctgt gagcggaggc     120
   cacacacgtg gaattcccga gagcgccgag gaggagggag acctggagca gcgtccccgg     180
   gtgggaaaag caggggtgag aaaagcagtg ggaggacgca gtcattgtct ccactgtctg     240
   cagggctgcg aggtcgagga gaagcaggtc gcatctctgt tcacccggag gaagtcatgg     300
   ggggacaagc ttggggcccc taaagcacag ggtcatggga accctgatcc tatacttcca     360
   gacctcctgt taggccttcc tctgaagcag agctcggtga agtacagccc gtggttcaaa     420
   tccagcgact gcctgttttt taagccaagt tttcgggcac gagccggtgc caggcccatt     480
   cgtttggttc ttgtctgtgg ctgctttttg cacgctggcg gcggagcgga ggggctggga     540
   cggacccttg gagaatgttc tgagcagcaa gctgggagtt ctcctgcggg aattctgaaa     600
   ggcccatccg aggtgtggcc ccctcagctt tcagtgcccg gcttgccccc cccacccccg     660
   ctcaaatcca cccttgcccc ccgaggctcc ccgtggaagg gctctgatat ctgcctgcac     720
   ctcattgcca ggcttgcctg cgctcctgca acaggcacct ttggtcttgg ggcagctgag     780
   gctcgtaggc tagctgaggg gctggacaag ccttacctgg caggctgtaa aacagttctg     840
   gaaggtcagg attattttca gcatgcctac gacttgagga aagcgtgccg gggagagctt     900
   agagaagtca ttcggggtca cgtcgtggct tattgtcgca accctgcacc tacatcagtg     960
   gtggcatcgt tagggacctt gggattgggc aacgagtctg gccccatgtg catgcgttgc    1020
   cgccagctgt gtacagatgt tccaagtgaa gggtgcaatc agagcagcct gtgcttccga    1080
   gtgcgagcag cccagagctg tgaggccaca gaagagggaa atccccatag agttcaagag    1140
   gatgtggcga ttgcttcata tttaatgtca gtgtcagtgc atggggttga tgatgtggtg    1200
   tctgaacatg tgctcgctgc cgtggcacca aaacgcaagg gcagacggtc agtctgccaa    1260
   ggcacgagta accggctcac ccagttgggc acttttgaag accactttct gagccttcag    1320
   aggatgttca ataactgtga ggtggtcctg gggaatttgg aaattaccta catgcaaagg    1380
   aattatgacc tttccttctt aaagaccatc caggaagttg ccgggtatgt attgattgcc    1440
   ctcaacacgg tggagaagat tcctttggaa aacctgcaga tcatccgagg aaatgtgctc    1500
   tatgaaaaca cccatgcctt atccgtcctc tccaactatg gctcaaataa aactggactg    1560
   caggagcttc ccctgagaaa cttgcatgaa atactacaag gcgctgtgcg cttcagcaat    1620
   aaccctgtcc tctgcaacgt ggaaaccatc cagtggcggg acatcgttga caacgatttt    1680
   ataagcaaca tgtcgatgga catccagaac caagcgggca ggtgccaaaa gtgtgatcca    1740
   agctgtccca atggaagttg ctggggtccg ggaaaggaga attgccagaa actgaccaaa    1800
   atcatctgtg cccagcagtg ttcggggcgc tgccgcggca ggtcccccag cgactgctgc    1860
   cacaaccagt gtgccgccgg ctgcacgggg ccccgcgaga gcgactgcct ggtctgccgc    1920
   aagttccggg atgaagccac gtgcaaggac acctgcccgc ccctcatgct ctacaacccc    1980
   accacctacc aaatggacgt caacccagag ggaaaataca gctttggtgc cacctgcgtg    2040
   aagaaatgcc cccgcaacta cgtggtgaca gaccacggtt catgtgtccg cgcctgcagc    2100
   tctgacagct acgaggtgga ggaggatggt gtccgcaagt gtaagaagtg tgaggggcct    2160
   tgtcgcaaag tttgtaatgg aatagggatt ggagagttca aagacacact ttccataaat    2220
   gctacaaaca ttaaacactt caaaaactgc acgtcgatca gtggagacct tcatatcttg    2280
   ccagtcgcat ttagaggtga ctccttcacg cataccctac ctctagatcc gaaggagctg     2340
   gatatcctga aaactgtcaa ggaaataaca gggtttttgc tgattcaggc ctggcccgaa    2400
   aataggactg acctccatgc tttcgagaac ctagaaatca tacgtggaag aacaaagcaa    2460
   catggccagt tttctctggc ggtcgtcggc ctgaacataa cgtccttggg attacgctcc     2520
   ctcaaggaga taagcgatgg agatgtgatc atctctggaa accgaaaatt gtgctatgca 2580
   aatacaataa actggaagaa actctttggg acttcaagtc agaaaaccaa aattataaac 2640
   aacaaagatg aaaaagcctg caaggccata ggccacgtct gccatccatt gtgctcctca 2700
   gaaggctgct ggggcccagg gccgagagac tgcgtgtcct gccgaaacgt cagccgtggc 2760
   aaggaatgtg tggaaaagtg caacattctg gagggggagc caagggagtt tgtggagaat 2820
   tcggagtgca tacagtgcca tccggaatgc ctgccccagg ccatgaacat aacctgcaca 2880
   ggacgggggc cggacagctg catcaagtgc gcccactaca tcgatggccc tcactgcgtc 2940
   aagacctgcc cggctggcat catgggagaa aacaacaccc tggtctggaa gttttcggat 3000
   ggcagccgca tgtgccacct gtgccatcca aactgcacct atggctgtga gggaccaggt 3060
   cttgaaggct gtgcaaaacc tgggcccaag atcccatcca ttgctaccgg gattgtcggc 3120
   ggcctcctct tggtggtggt ggtggccctt ggagtgggcc tctttttgcg ccggcgccac 3180
   attgtccgga agcgcacgct tcgcagactg ctgcaagaaa gagagcttgt cgagcctctt 3240
   acacccagcg gagaagctcc caaccaggct ctcttgagga tcttaaagga gacggagttc 3300
   aaaaagatca aggtgctggg ctctggagca ttcggcacag tgtacaaggg actctggatc 3360
   ccagaaggcg agaaggttaa aattcccgtg gccatcaagg aattgagaga agccacgtct 3420
   cccaaagcca acaaggaaat tcttgatgaa gcctacgtga tggccagtgt ggacaatccc 3480
   cacgtgtgcc gcctcctggg catctgcctg acgtccacgg tgcagctcat cacgcagctc 3540
   atgccctttg gctgcctcct ggactatgtc cgcgagcaca aggacaacat cggctcccag 3600
   cacctgctca actggtgtgt gcagattgca aagggcatga actacctgga agaccggcgc 3660
   ttggtgcacc gcgacctggc ggccaggaac gtcctggtga agaccccgca gcacgtcaag 3720
   atcacagatt ttgggctggc caaactgctg ggtgccgagg agaaagagta ccacgcggaa 3780
   ggaggcaaag tgcctatcaa gtggatggct ttagaatcga ttttacaccg aatttatacc 3840
   caccaaagcg atgtgtggag ctacggcgtc accgtgtggg agctgatgac cttcgggtcc 3900
   aagccttacg acggtatccc tgcaagtgag atctccacca tcctggagaa gggagagcgc 3960
   ctcccgcagc cgcccatatg caccatcgat gtctacatga tcatggtcaa gtgctggatg 4020
   atagatgcag acagtcgccc aaaattccgt gagttgatca tcgaattctc caaaatggcc 4080
   cgagacccgc agcgctacct tgtcatccag ggagatgaga ggatgcattt gccaagccct 4140
   acagactcca atttttaccg cgccctgatg gacgaggagg acatggagga tgttgtggat 4200
   gctgacgagt acctcatccc ccagcagggc ttcttccaca gcccctccac ttcccggacc 4260
   cccctcctaa gttctctgag cgccaccagc aacagttcca acgtggcttg catcgaccga 4320
   aatgggacct gtcccctcaa agaagacagc ttcttgcagc ggtacagctc agaccccact 4380
   ggcaccttga cggaggacaa catagatgac actttcctcc cagcacccga atacataaac 4440
   cagtctgttc ccaaaaggcc tgcgggttct gtccagaatc ccgtctatca caatcagcct 4500
   ctaaatccag ctcctgccag agaccctcac taccaaaatc cccacagcaa cgcagtggac     4560
   aaccctgagt atctcaacac ccaccccacc tgcgtcaaca gtgtcctcga caggcccagc     4620
   ctctggaccc aggaggccaa ccaccaaatc agcctggaca accctgacta ccagcaggac     4680
   ttctttccca aggaagccaa gtccaatggc atttttaagg gccctgcagc tgaaaatgca     4740
   gactacctga gggtagcgcc accaagcagt gagtttattg gagcgtga                 4788

## Description

### FIELD OF THE INVENTION

The invention relates to the fields of diagnostics and therapeutics, in particular to a method of providing personalized management to brain cancer patients based on the expression of certain genes in a sample from said patients, which certain serve as treatment targets.

### BACKGROUND OF THE INVENTION

### Gliomas: Diagnosis and disease categorization

A glioma is a type of cancer that starts in the brain or spine. It is called a glioma because it arises from glial cells and/or its precursors. The most common site of gliomas is the brain. Gliomas are classified by cell type, grade, and location. Gliomas are named according to the specific type of cell they most closely resemble. The main types of gliomas are:
- Ependymomas, gliomas derived from ependymal cells.
- Astrocytomas, gliomas derived from astrocytes; the glioblastoma multiforme (GBM) is the most common astrocytoma.
- Oligodendrogliomas, gliomas derived from oligodendrocytes.
- Mixed gliomas, such as oligoastrocytomas, that contain cells from different types of glia.

Gliomas are further categorized according to their grade, which is determined by pathologic evaluation of the tumor. Thus we can distinguish between low-grade gliomas that are well-differentiated (not anaplastic), benign and portend a better prognosis for the patient; and high-grade gliomas, that are undifferentiated or anaplastic, malignant and carry a worse prognosis.

Of numerous grading systems in use, the most common is the World Health Organization (WHO) grading system for astrocytoma.

### Treatment of brain gliomas

The treatment for brain gliomas depends on the location, the cell type and the grade of malignancy. Often, treatment is a combined approach, using surgery, radiation therapy, and chemotherapy. The radiation therapy is in the form of external beam radiation or the stereotactic approach using radiosurgery. Spinal cord tumors can be treated by surgery and radiation. Temozolomide is a chemotherapeutic drug that is able to cross the blood-brain barrier effectively and is being used in therapy. Despite these approaches most high grade glioma patients succumb to their disease. New therapeutic interventions to critical targets are needed to improve outcome in this patient population.

### Glioblastoma multiforme (GBM)

The glioblastoma multiforme (GBM, WHO grade IV) is a highly aggressive brain tumor presenting as one of two subtypes with distinct clinical histories and molecular profiles. The primary GBM presents acutely as a high-grade disease and the secondary GBM subtype evolves from the slow progression of a low-grade disease.

Brown et al. (J Clin Oncology. 2008, 5603-5609) describe a phase I/II trial of erlotinib combined with temozolomide in patients suffering GBM. These authors tried to correlate the response of the patients with several molecular markers like EGFR, PTEN, P53, etc., but failed to observe any correlation between survival and expression levels of said genes.

Mirimanoff et al. (J Clin Oncology. 2006, 2563-2569) describe a completed EORTC Phase III trial, where the MGMT promoter methylation was the strongest predictor for outcome and positive response to temozolomide.

Van den Bent et al. (J Clin Oncology. 2009, 27:1268-1274) describe a recent randomized Phase II EORTC trial. Patients with progressive GBM after prior radiotherapy were randomly assigned to either erlotinib or a control arm that received treatment with either temozolomide or carmustine (BCNU). The primary end point was 6-month progression-free survival (PFS). Tumor specimens obtained at first surgery were investigated for EGFR expression; EGFRvIII mutants; EGFR amplification; EGFR mutations in exons 18, 19, and 21; and pAkt. No clear biomarker associated with improved outcome to erlotinib was identified.

Smith et al. (J. National Cancer Institute. 2001, 1246-1256) describe methods for predicting the survival of patients with anaplastic astrocytoma and GBM. These authors identify that mutation of PTEN and EGFR amplification are independent prognostic markers for patients with anaplastic astrocytoma and EGFR amplification is a survival marker for older patients with GBM.

Umesh et al. (Clinical Neuropathology. Vol. 28 - No. 5/2009 (362-372)) describe a method for predicting the patient outcome of glioma comprising the detection of EGFR amplification and PTEN LOH by immunohistochemistry. The conclusion described in said document is that EGFR amplification associated with LOH of the PTEN gene is a trend to poor survival.

Prados et al. (J Clin Oncology. 2009, 27(4):579-84) describe a Phase II trial which evaluated erlotinib plus temozolomide during and after radiation, patients treated with combination therapy (i.e., erlotinib and temozolomide) had better survival. In addition, the study also evaluated several biomarkers and found that methylation of the MGMT promoter along with PTEN expression was associated with improved survival.

However, there is still a need for further markers and markers combinations useful for predicting the clinical outcome of the glioma patients. A special area for diagnosis and prognosis is the study of the biopsy sample. An integrated approach able to better define patient outcome based on the most appropriate treatment using tumor profiles will be critical, offering in addition to the glioma patient a better quality of life.

### SUMMARY OF THE INVENTION

In an aspect, the invention relates to a method for predicting the clinical outcome of a subject suffering from glioma which comprises determining the expression level of the EGFR gene or the polysomy / amplification levels of the EGFR locus on chromosome 7 and the loss of heterozygosity (LOH) levels of the PTEN gene in a sample from the same subject, and comparing said expression level or the polysomy / amplification levels of the EGFR gene and the LOH level of the PTEN gene with standard reference values, wherein the LOH level of the PTEN gene is measured by PCR, by a hybridization-based assay, by sequencing, or by SNP analysis; and wherein a high LOH level of the PTEN gene with respect to said standard reference value and a high expression level and/or high levels of polysomy / amplification of the EGFR gene with respect to said standard reference values are indicative of a good clinical outcome of the subject.

In another aspect is also disclosed a method for predicting the clinical outcome of a subject suffering from glioma that comprises determining the LOH level of the PTEN gene in a sample from the subject, and comparing said LOH level of PTEN gene with a standard reference value, wherein the LOH level of the PTEN gene is measured by PCR, by a hybridization-based assay, by sequencing, or by SNP analysis ; and wherein a high LOH level of the PTEN gene with respect to said standard reference value, is indicative of a bad clinical outcome of the subject.

In another aspect, the invention relates to the use of a kit comprising agents capable of specifically detecting the expression level and/or the polysomy / amplification of the EGFR gene and the LOH of the PTEN gene and, optionally, a reagent for detecting a housekeeping gene or the protein encoded by said housekeeping gene and/or a reagent for detecting the chromosomes 7 and 10, for predicting the clinical outcome of a subject suffering from glioblastoma multiforme, wherein the set of agents capable of specifically determining the LOH level of the PTEN gene comprises a pair of oligonucleotide primers suitable for amplifying a specific fragment of the PTEN gene or an optionally labeled oligonucleotide probe which selectively binds to a target polynucleotide sequence on the chromosome region of the PTEN gene or reagents suitable for performing a sequencing reaction or reagents for performing a SNP analysis, and wherein if said agents detect a high expression levels or a high level of polysomy/amplification of the EGFR gene and a high LOH level of the PTEN gene, with respect to reference values, then the clinical outcome of the subject is good.

In another aspect, it is also disclosed the use of erlotinib and/or temozolomide in the manufacture of a medicament for the treatment of a glioma in a subject suffering from a glioma, wherein the medicament is for a subject having a high LOH level of the PTEN gene, as measured by PCR, by a hybridization-based assay, by sequencing, or by an SNP analysis, with respect to a standard reference value and high expression levels and/or high polysomy / amplification of the EGFR gene with respect to standard reference values.

It is also disclosed the use of radiotherapy in a regime for the treatment of a glioma in a subject suffering from a glioma, wherein said subject has a high LOH level of the PTEN gene, as measured by PCR, by a hybridization-based assay, by a sequencing technology, or by a SNP analysis, with respect to a standard reference value and high expression levels and/or high polysomy / amplification of the EGFR gene with respect to standard reference values.

### BRIEF DESCRIPTION OF THE DRAWINGS

Figure 1. Kaplan-Meier survival curve illustrating time from first surgery to death / end-of follow-up of only (Pure) GBM patients, astrocyoma patients and oligodendroglioma patients.
Figure 2. Kaplan-Meier curve estimating survival from first surgery to death / recurrence / progression or end-of follow-up of only (Pure) GBM patients, astrocyoma patients and oligodendroglioma patients.
Figure 3. Kaplan-Meier survival curve demonstrating stratification of patients in the glioblastoma multiforme (GBM), anaplastic astrocytoma and mixed group with PTEN LOH (p=0.04).
Figure 4. Kaplan-Meier survival curve demonstrating stratification of patients in the glioblastoma multiforme (GBM), anaplastic astrocytoma and mixed group as having both EGFR AMP/HP (amplification - high polysomy) and PTEN LOH (p=0.034).
Figure 5. Representative images of glioblastoma multiforme (GBM) specimens stained with H&E (Hematoxylin and Eosin) (A). Example of FISH experiments where AMP/HP EGFR DNA FISH (B) and monosomy PTEN (C) are illustrated.

### DETAILED DESCRIPTION OF THE INVENTION

In order to facilitate the understanding of the invention described in this patent application, the meaning of some terms and expressions in the context of the invention are explained below.

The term "subject" refers to a member of a mammal animal species, and includes, but is not limited thereto, domestic animals, primates and humans; the subject is preferably a human being, male or female, of any age or race. Alternatively, the term "individual" is also sometimes used in this description to refer to human beings.

The term "protein" refers to a molecular chain of amino acids, linked by covalent or non-covalent bonds. The term includes all the forms of post-translational modifications, for example, glycosylation, phosphorylation or acetylation.

The term "antibody" refers to a protein with the capacity to specifically bind to an antigen. The term antibody comprises recombinant antibodies, monoclonal antibodies, or polyclonal antibodies, intact, or fragments thereof which maintain the capacity to bind to the antigen, combibodies, etc., both human or humanised and of non-human origin.

The term "primer oligonucleotide", as used in the present invention, refers to a nucleotide sequence, which is complementary to a nucleotide sequence of a selected gene. Each primer oligonucleotide hybridises with its target nucleotide sequence and acts as an initiation point for DNA polymerisation.

The inventors of the present invention have found that the clinical outcome of patients suffering from glioma cancer correlates with expression levels and/or the polysomy / amplification levels of the EGFR gene and with the LOH level of the PTEN gene.

Thus, in an aspect, the invention relates to a method for predicting the clinical outcome of a subject suffering from glioma, hereinafter referred to as method [1] of the invention, that comprises:
a) determining the expression level or the polysomy / amplification level of the EGFR gene and the LOH level of the PTEN gene in a sample from the same subject, and
b) comparing said expression level or the polysomy / amplification level of the EGFR gene and the LOH level of the PTEN gene with standard reference values,
   wherein the LOH level of the PTEN gene is measured by PCR, by a hybridization-based assay, by sequencing, or by a SNP analysis; and
   wherein a high LOH level of the PTEN gene with respect to said standard reference value and a high expression level and/or high level of polysomy / amplification of the EGFR gene with respect to said standard reference values are indicative of a good clinical outcome of the subject.

In the present invention a "glioma" is a type of cancer that starts in the brain or spine. It is called a glioma because it arises from glial cells and/or its precursors. The most common site of gliomas is the brain. Gliomas are classified by cell type, grade, and location. Gliomas are named according to the specific type of cell they most closely resemble. The main types of gliomas are:
- Ependymomas, gliomas derived from ependymal cells.
- Astrocytomas, gliomas derived from astrocytes; the glioblastoma multiforme (GBM) is the most common astrocytoma.
- Oligodendrogliomas, gliomas derived from oligodendrocytes.
- Mixed gliomas, such as oligoastrocytomas, that contain cells from different types of glia.

Gliomas are further categorized according to their grade, which is determined by pathologic evaluation of the tumor. Thus, one can distinguish between (i) low-grade gliomas that are well-differentiated (not anaplastic), benign and portend a better prognosis for the patient; and (ii) high-grade gliomas, that are undifferentiated or anaplastic, malignant and carry a worse prognosis.

In a preferred embodiment, the glioma is a gliobastoma multiforme (GBM) and more preferably the gliobastoma is an early gliobastoma.

The gliobastoma multiforme (GBM) is the most common and malignant form of glial tumors and is composed of a heterogenous mixture of poorly differentiated malignant astrocytes and dysplastic endothelial cells. It primarily affects adults, involves the cerebral hemispheres and has a rapid disease course which often leads to death.

In the present invention "clinical outcome" is understood as the expected course of a disease. It denotes the doctor's prediction of how a subject's disease will progress, and whether there is chance of recovery or recurrence. The prediction of the clinical outcome can be done by using any endpoint measurements used in oncology and known to the skilled practitioner. Useful endpoint parameters to describe the evolution of a disease include:
- disease-free progression which, as used herein, describes the proportion of patients in complete remission who have had no recurrence of disease during the time period under study;
- objective response, which, as used herein, describes the proportion of treated people in whom a complete or partial response is observed;
- tumor control, which, as used herein, relates to the proportion of treated people in whom complete response, partial response, minor response or stable disease in equal to or more than (≥) 6 months is observed;
- progression free survival which, as used herein, is defined as the time from start of treatment to the first measurement of cancer growth;
- six-month progression free survival or "PFS6" rate which, as used herein, relates to the percentage of people wherein free of progression in the first six months after the initiation of the therapy; and
- median survival which, as used herein, relates to the time at which half of the patients enrolled in the study are still alive.

A good clinical outcome is understood as a situation where at least 10%, 20%, 30%, 40%, 50%, 60%, 70%, 80%, 90%, 95% or even more of the patients have a positive result regarding the endpoint parameters described above.

The term "sample" as used herein, relates to any sample which can be obtained from the patient. The present method can be applied to any type of biological sample from a patient, such as a biopsy sample, tissue, cell or fluid (whole blood, serum, saliva, semen, sputum, urine, cerebral spinal fluid (CSF), tears, mucus, sweat, milk, brain extracts and the like). In a particular embodiment, said sample is a tumour tissue sample or portion thereof. In a more particular embodiment, said tumor tissue sample is a brain tumor tissue sample from a patient suffering from brain cancer. Said sample can be obtained by conventional methods, e.g., biopsy, by using methods well known to those of ordinary skill in the related medical arts. Methods for obtaining the sample from the biopsy include gross apportioning of a mass, or microdissection or other art-known cell-separation methods. Tumour cells can additionally be obtained from fine needle aspiration cytology. In order to simplify conservation and handling of the samples, these can be formalin-fixed and paraffin-embedded or first frozen and then embedded in a cryosolidifiable medium, such as OCT-Compound, through immersion in a highly cryogenic medium that allows for rapid freeze. In a particular embodiment, the sample is a tumor sample that contains a substantially number of tumor cells.

The samples may be obtained from subjects previously diagnosed with glioma (patients), or from subjects who have not been previously diagnosed with glioma, or from patients diagnosed with glioma who are undergoing treatment, or from patients diagnosed with glioma who have been previously treated.

PTEN is the phosphatase and tensin homolog protein also known as BZS; MHAM; TEP1; MMAC1; PTEN1; 10q23del or MGC11227. PTEN is a protein, which in humans is encoded by the PTEN gene (RefSEq ID NM_000314 SEQ ID NO: 1, Protein reference NP_000305.3 SEQ ID NO: 2) (Steck PA, et al. 1997 Nat. Genet. 15 (4): 356-62 2). PTEN acts as a tumor suppressor gene through the action of its phosphatase protein product. This phosphatase is involved in the regulation of the cell cycle, preventing cells from growing and dividing too rapidly. Mutations of this gene contribute to the development of certain cancers (Chu EC, et al. 2004 Med. Sci. Monit. 10 (10): RA235-41 3). It does exist as homologues in other species, such as mice (NM_008960.2, SEQ ID NO: 3), rat (NM_031606.1, SEQ ID NO: 4), dog (NM_001003192, SEQ ID NO: 5), etc.

The protein encoded by the PTEN gene is a phosphatidylinositol-3,4,5-trisphosphate-3-phosphatase. It contains a tensin like domain as well as a catalytic domain similar to that of the dual specificity protein tyrosine phosphatases. Unlike most of the protein tyrosine phosphatases, this protein preferentially dephosphorylates phosphoinositide substrates. It negatively regulates intracellular levels of phosphatidylinositol-3,4,5-trisphosphate in cells and functions as a tumor suppressor by negatively regulating Akt/PKB signaling pathway (Hamada K, et al 2005 Genes Dev 19 (17): 2054-65).

The epidermal growth factor receptor (EGFR; ErbB-1; HER1 in humans) is the cell-surface receptor for members of the epidermal growth factor family (EGF-family) of extracellular protein ligands (Herbst RS (2004). Int. J. Radiat. Oncol. Biol. Phys. 59 (2 Suppl): 21-6. 1) The EGFR is a member of the ErbB family of receptors. The EGFR is a protein which in humans is encoded by different isoforms: EGFR transcript variant 1(NM_005228.3, SEQ ID NO: 6), transcript variant 2 (NM_201282.1, SEQ ID NO: 7), transcript variant 3 (NM_201283.1, SEQ ID NO: 8) and transcript variant 4 (NM_201284.1, SEQ ID NO: 9). It does exist as homologues in other species, such as mice (NM_207655.2, SEQ ID NO: 10 and NM_007912.4, SEQ ID NO: 11), rat (NM_031507.1, SEQ ID NO: 12), dog (XM_533073.2, SEQ ID NO: 13), etc.

The method [1] of the invention comprises determining the expression level of the EGFR gene. As the person skilled in the art understands, the expression level of a gene can be determined by measuring the levels of mRNA encoded by said gene, by measuring both the levels of proteins encoded by said gene and the levels of variants thereof, by the use of surrogates (DNA copy number) for associating gene level with mRNA and protein product of said gene, etc.

A variant of a protein, e.g., EGFR or PTEN, as used herein may be (i) a protein in which one or more of the amino acid residues is/are substituted with a conserved or non-conserved amino acid residue (preferably a conserved amino acid residue) and such substituted amino acid residue may or may not be one encoded by the genetic code, (ii) a protein having one or more modified amino acid residues, e.g., residues that are modified by the attachment of substituent groups, (iii) a modified protein said protein being the results of an alternative splicing of the mRNA encoding the EGFR or PTEN protein, and/or (iv) a fragment of the protein. The term "fragment" includes also a peptide or protein generated via proteolytic cleavage (including multi-site proteolysis) of an original protein. Variants are deemed to be within the scope of those skilled in the art from the teaching herein.

As known in the art the "similarity" between two proteins is determined by comparing the amino acid sequence and its conserved amino acid substitutes of one protein to a sequence of a second protein. Variants according to the present invention include peptides or protein having amino acid sequences that are at least 60%, 65%, 70%, 72%, 74%, 76%, 78%, 80%, 82%, 84%, 86%, 88%, 90%, 95%, or even more, similar or identical to the original amino acid sequence. The degree of identity between two proteins can be determined using computer algorithms and methods that are widely known for the persons skilled in the art. The identity between two amino acid sequences is preferably determined by using the BLASTP algorithm (BLASTManual, Altschul, S., et al., NCBI NLM NIH Bethesda, Md. 20894, Altschul, S., et al., J. Mol. Biol. 215: 403-410 (1990)).

The proteins can be post-translationally modified. For example, post-translational modifications that fall within the scope of the present invention include signal peptide cleavage, glycosylation, acetylation, isoprenylation, proteolysis myristoylation, protein folding and proteolytic processing, etc. Additionally, the proteins may include unnatural amino acids formed by post-translational modification or by introducing unnatural amino acids during translation.

In a preferred embodiment, the determination of the expression levels of the EGFR gene can be carried out by measuring the expression level of the mRNA encoded by the EGFR gene, respectively. For this purpose, the biological sample may be treated to physically or mechanically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods known in the art, for example, Sambrook, Fischer and Maniatis, Molecular Cloning, a laboratory manual, (2nd ed.), Cold Spring Harbor Laboratory Press, New York, (1989). Preferably, care is taken to avoid degradation of the RNA during the extraction process.

In a particular embodiment, the expression level is determined by using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene, for example.

Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample.

While all techniques of gene expression profiling (RT-PCR, SAGE, or TaqMan) are suitable for use in performing the foregoing aspects of the invention, the expression levels of the mRNA coding for EGFR or for PTEN are often determined by reverse transcription polymerase chain reaction (RT-PCR). The detection can be carried out in individual samples or in tissue microarrays.

In order to normalize the values of the mRNA expression among the different samples, it is possible to compare the expression levels of the mRNA of interest in the test samples with the expression of a control RNA. A "control RNA" as used herein, relates to a RNA whose expression levels do not change or change only in limited amounts in tumor cells with respect to non-tumorigenic cells. Preferably, a control RNA is a mRNA derived from a housekeeping gene and which code for a protein which is constitutively expressed and carry out essential cellular functions. Preferred housekeeping genes for use in the present invention include β-2-microglobulin, ubiquitin, 18-S ribosomal protein, cyclophilin, GAPDH and actin. In a preferred embodiment, the control RNA is β-actin mRNA. In an embodiment relative gene expression quantification is calculated according to the comparative Ct method using β-action as an endogenous control and commercial RNA controls as calibrators. Final results are determined according to the formula 2-(ΔCt sample-ΔCt calibrator), wherein ΔCT values of the calibrator and sample are determined by subtracting the CT value of the target gene from the value of the β-action gene.

The determination of the level of expression of the EGFR gene needs to be correlated with the standard reference values. Standard reference values correspond to the median value of the expression levels of the EGFR gene measured in a collection of samples from healthy patients. Once this median value is established, the level of this marker expressed in tumor tissues from patients can be compared with this median value, and thus be assigned a level of "low", "normal" or "high". The collection of samples from which the reference level is derived will preferably be constituted from healthy persons from the same age as the patients. In any case it can contain a different number of samples. In a more preferred embodiment, the samples are biopsy brain samples. Preferably the collection should be sufficient to provide an accurate standard reference value. Typically, the number of samples used for determining a standard reference value is at least 10, preferably more than 10, 20, 30, 40, 50, 60, 70, 80, 90, 100, 150, 200, 500 or even more samples. The standard can also include 'normal' cells present within the diseased and / or cancerous tissue. This is particularly true for brain tumor resection and occasionally biopsy specimens which typically contain a rim of normal tissue or have inflammatory cells etc, which do not contain gene changes. In addition, it can be used a cell culture system to evaluate gene content, as a way to determine the presence or loss of individual genes.

In a particular embodiment, an increase in the expression of the EGFR gene, , as determined in the sample above the standard reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as a "high" expression level of the EGFR gene. In another particular embodiment, a decrease in the expression of the EGFR gene, as determined in the sample below the standard reference value of at least 0.9-fold, 0.75-fold, 0.2-fold, 0.1-fold, 0.05-fold, 0.025-fold, 0.02-fold, 0.01-fold, 0.005-fold or even less compared with the standard reference value is considered as a "low" expression level of the EGFR gene.

Alternatively, in another particular embodiment, the expression level of the EGFR gene can be determined by measuring both the level of the protein encoded by said gene, i.e. EGFR protein, and the levels of a variant thereof. Although it would also be possible to determine the expression level of the PTEN gene by measuring both the level of the protein encoded by said gene, i.e. PTEN protein, and the levels of a variant thereof, in practice, said option is not suitable for performing the teachings of the instant invention as will be discussed below. The skilled person in the art knows that loss of protein expression may also be the result of methylation of the gene promoter and not a loss of the gene per se which impacts on other associated genes.

The determination of the expression level of a protein, e.g., EGFR protein, or a variant thereof can be carried out by any conventional technique known for the skilled person in the art. In a particular embodiment, the determination of the expression levels of said protein, e.g., EGFR protein, or a variant thereof, is carried out by immunological techniques such as e.g., ELISA (Enzyma-Linked ImmuneSorbent Assay), Western blot, immunofluorescence (IF), immunohistochemistry (IHC) analysis, etc. ELISA is based on the use of antigens or antibodies labeled (e.g., with enzymes) so that the conjugates formed between the target antigen and the labeled antibody results in the formation of, e.g., enzymatically-active complexes. Since one of the components (the antigen or the labelled antibody) is immobilised on a support, the antibody-antigen complexes are immobilised on the support and thus, it can be detected by the addition of a substrate which is converted by the enzyme to a product which is detectable by, e.g. spectrophotometry, fluorometry, etc. This technique does not allow the exact localisation of the target protein or the determination of its molecular weight but allows a very specific and highly sensitive detection of the target protein in a variety of biological samples (serum, plasma, tissue homogenates, postnuclear supernatants, ascites and the like). Western blot is based on the detection of a protein previously resolved by gel electrophoresis under denaturing conditions and immobilized on a membrane, generally nitrocellulose, by incubation with an antibody specific to said protein and a developing system (e.g. chemo-luminiscent, etc.). The analysis by immunofluorescence (IF) requires the use of an antibody specific for the target protein for the analysis of the expression and subcellular localization by microscopy. Generally, the cells under study are previously fixed with paraformaldehyde and permeabilised with a non-ionic detergent. In a preferred embodiment, the EGFR protein is detected by an immunohistochemistry (IHC) analysis using thin sections of the biological sample immobilised on coated slides. The sections are then deparaffinised, if derived from a paraffinised tissue sample, and treated so as to retrieve the antigen. The detection can be carried out in individual samples or in tissue microarrays. In another embodiment the method used for determining the expression level of a protein, e.g., EGFR protein, or a variant thereof is a proteomic array.

Practically any antibody or reagent known to bind with high affinity to the target protein can be used for detecting the amount of said target protein. It is preferred nevertheless the use of an antibody, for example polyclonal sera, hybridoma supernatants or monoclonal antibodies, antibody fragments, Fv, Fab, Fab' y F(ab')2, ScFv, diabodies, triabodies, tetrabodies and humanised antibodies.

In yet another embodiment, the determination of the expression level of a protein, e.g., EGFR, etc., can be carried out by constructing a tissue microarray (TMA) containing the patient samples assembled, and determining the expression levels of said protein by IHC techniques. Immunostaining intensity can be evaluated by two different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, as low expression (1+) versus moderate (2+) expression and as high (3+) expression, taking into account the expression in tumoral cells and the specific cut-off for each marker. As a general criterion, the cut-offs were selected in order to facilitate reproducibility, and when possible, to translate biological events.

The determination of the expression level of the EGFR gene needs to be correlated with the standard reference values which correspond to the median value of expression levels of the EGFR gene measured in a collection of brain tissue samples from healthy patients. Preferably the collection should be sufficient to provide an accurate reference level. Typically the number of samples used for determining the standard reference values is at least 10, preferebly more than 10, 20, 30, 40, 50, 60, 70, 80, 90, 100, 150, 200, 500, or even more, samples.

In a preferred embodiment the sample is a biopsy. Once this median value is established, the level of this marker expressed in tumor tissues from patients can be compared with this median value, and thus be assigned a level of "low", "normal" or "high" as defined above.

"Polysomy" / "amplification" of the EGFR locus on chromosome 7 as used in the present invention is understood as the presence of more than one copy of the EGFR locus on the chromosome 7.

Further, as used herein, the term "LOH" within the context of the present invention refers to "loss of heterozygosity". LOH in a cell represents the loss of normal function of one allele of a gene in which the other allele was already inactivated. This term is mostly used in the context of oncogenesis; after an inactivating mutation in one allele of a tumor suppressor gene occurs in the parent's germline cell, it is passed on to the zygote resulting in an offspring that is heterozygous for that allele. In oncology, loss of heterozygosity (LOH) occurs when the remaining functional allele in a somatic cell of the offspring becomes inactivated by mutation. In the case of PTEN LOH, this results in no normal tumor suppressor being produced.

In a particular embodiment, the determination of the polysomy / amplification level of the EGFR gene and the determination of the LOH levels of the PTEN gene can be measured, for example, in the DNA obtained from the tumor cells according to standard procedures such as, for example, quantitative PCR, comparative genomic hybridization (CGH) to microarray technologies, etc.; or in the tumor cells from the paraffined-embedded section or from the cytology preparation by FISH using appropriate molecular probes, etc.

In a particular embodiment, the detection of the polysomy / amplification level of the EGFR gene and the determination of the LOH levels of the PTEN gene is carried out by a polymerase chain reaction (PCR).

In a particular embodiment, the detection of the polysomy / amplification level of the EGFR gene and the determination of the LOH levels of the PTEN gene is carried out by a hybridization-based assay. In a particular embodiment, the detecting step of the method [1] of the invention comprises contacting the nucleic acid sample with one or more nucleic acid probes each of which selectively binds to a target polynucleotide sequence on the chromosome region of the EGFR or PTEN loci, under conditions in which the probe forms a stable hybridization complex with the target polynucleotide sequence; and detecting the hybridization complex. In a particular embodiment, the nucleic acid probes used in the method [1] of the invention are labeled with a fluorophore. Alternatively, in another particular embodiment, the step of detecting the hybridization complex comprises determining the copy number of the target polynucleotide sequence, thereby determining the presence of polysomy or LOH of the target gene.

In a preferred embodiment, said hybridization-based assay is selected from the group consisting of Southern blot, *in situ* hybridization (ISH), fluorescence ISH (FISH) and comparative genomic hybridization (CGH). Southern blot is a method routinely used in molecular biology for detection of a specific DNA sequence in DNA samples; Southern blotting generally combines transfer of electrophoresis-separated DNA fragments to a filter membrane and subsequent fragment detection by probe hybridization. *In situ* hybridization (ISH) is a type of hybridization that uses a labeled complementary DNA or RNA strand (i.e., probe) to localize a specific DNA or RNA sequence in a portion or section of tissue (*in situ*), or, if the tissue is small enough, in the entire tissue; DNA ISH can be used to determine the structure of chromosomes. *Fluorescence in situ* hybridization (FISH) is a cytogenetic technique used to detect and localize the presence or absence of specific DNA sequences on chromosomes. FISH uses fluorescent probes that bind to only those parts of the chromosome with which they show a high degree of sequence similarity. Fluorescence microscopy can be used to find out where the fluorescent probe bound to the chromosomes. FISH is often used for finding specific features in DNA for use in genetic counselling, medicine (for example, in medical diagnostics to assess chromosomal integrity), and species identification; FISH can also be used to detect and localize specific mRNAs and other transcripts within tissue sections or whole mounts (so, it can help define the spatial-temporal patterns of gene expression within cells and tissues). Comparative genomic hybridization (CGH) or Chromosomal Microarray Analysis (CMA) is a molecular-cytogenetic method for the analysis of copy number changes (gains/losses) in the DNA content of a given subject's DNA and often in tumor cells; CGH detects only unbalanced chromosomal changes. In a particularly preferred embodiment, said hybridization-based assay is a CGH assay.

In a particular embodiment, said hybridization-based assay is an array-based assay. In a particular embodiment, once the sample has been obtained and the total DNA has been extracted, genome-wide analysis of DNA copy number changes by CGH is carried out. In general, for a typical CGH measurement, total genomic DNA is isolated from test and reference cell populations, differentially labeled and hybridized to a representation of the genome that allows the binding of sequences at different genomic locations to be distinguished. Hybridization reactions can be performed under conditions of different stringency. The stringency of a hybridization reaction includes the difficulty with which any two nucleic acid molecules will hybridize to one another. Preferably, each hybridizing polynucleotide hybridizes to its corresponding polynucleotide under reduced stringency conditions, more preferably stringent conditions, and most preferably highly stringent conditions.

The amount of specimen DNA is frequently a constraint on CGH measurements. Typical array CGH procedures use from 300 ng to 3 µg of specimen DNA in the labeling reaction, equivalent to approximately 50.000 to 500.000 mammalian cells. Usually, random primer labeling protocols are employed, which also amplify the DNA, so that several micrograms (µg) are used in the hybridization.

Array CGH has been implemented using a wide variety of techniques. In a particular embodiment, array CGH is carried out using arrays from large-insert genomic clones such as bacterial artificial chromosomes (BACs). The general principles and conditions for detection of nucleic acids, such as using array CGH to BAC microarrays are well known for the skilled person in the art. This technique allows scanning the entire genome for DNA copy number changes therefore allowing quantitative detection of DNA copy number variation in tumor genomes with high resolution (Pinkel D, et al. Nat Genet 1998; 20(2):207-11; Hodgson G, et al. Nat Genet 2001;29(4):459-64). As an illustrative non-limitative example, in the array CGH carried out by the method [1] of the invention test tumor and reference genomic DNAs can be labeled by random priming using Cy3 and Cy5 fluorophores. Then, the images of the arrays may be analysed using, for example, a charge-coupled device (CCD) camera and appropriate software.

The major technical challenge of array CGH is generating hybridization signals that are sufficiently intense and specific so that copy number changes can be detected. The signal intensity on an array element is affected by a number of factors including the base composition, the proportion of repetitive sequence content, and the amount of DNA in the array element available for hybridization.

Array elements made from genomic BAC clones typically provide more intense signals than elements employing shorter sequences such as cDNAs, PCR products, and oligonucleotides. The higher signals from the more complex array elements result in better measurement precision, allowing detection of single-copy transition boundaries-even in specimens with a high proportion of normal cells.

In another preferred embodiment, said hybridization-based assay is a fluorescence *in situ* hybridization (FISH) or FISH plus spectral karotype (SKY) (Liehr T. et al 2008 Fluorescence In Situ Hybridization (FISH) - Application Guide, Springer Berlin Heidelberg).

FISH allows to detect and localize the presence or absence of specific DNA sequences on chromosomes, for example, FISH allows localize the signal to a specific (tumor) cell type. FISH uses fluorescent probes that bind to only those parts of the chromosome with which they show a high degree of sequence similarity. Fluorescence microscopy can be used to find out where the fluorescent probe bound to the chromosomes.

The term "probe" as used herein refers to any ribopolynucleotide or desoxiribopolynucleotide sequence that specifically binds to only those parts of the chromosome with which they show a high degree of sequence similarity. The probe must be large enough to hybridize specifically with its target but not so large as to impede the hybridization process. There are many different FISH probes that can be used in the present invention; illustrative, non-limitative examples thereof include bacterial artificial chromosomes (BACs), Tiling Oligonucleotide Probes (TOPs), etc. The design of FISH probes is well know for a person skilled in the art (please see Bayani J, Squire JA. Curr Protoc Cell Biol. 2004 Sep; Chapter 22: Unit 22.4; Bayani J, Squire J.Curr Protoc Cell Biol. 2004 Oct; Chapter 22: Unit 22.5; Navin, N. et al. Bioinformatics, Volume 22, Number 19, 1 October 2006 , pp. 2437-2438(2)) Publisher: Oxford University Press). The probe can be tagged directly with fluorophores, with targets for antibodies, with biotin, etc. Tagging can be done in various ways, such as by nick translation, by PCR using tagged nucleotides, etc.

The sample can be fixed and in paraffin embedded, thus an additionally step of deparafination may be performed.

For hybridization, an interphase or metaphase chromosome preparation is produced. The chromosomes are firmly attached to a substrate, usually, a glass. Repetitive DNA sequences must be blocked by adding short fragments of DNA to the sample. The probe is then applied to the chromosome DNA and incubated for approximately 12 hours while hybridizing. Several wash steps remove all unhybridized or partially-hybridized probes. After standard post hybridization washes the slides are stained with the DNA staining probe such DAPI and mounted with a mounting agent such as antifade.

The results are then visualized and quantified by using, for example, a microscope that is capable of exciting the dye and recording images. If the fluorescent signal is weak, amplification of the signal may be necessary in order to exceed the detection threshold of the microscope. Fluorescent signal strength depends on many factors such as probe labeling efficiency, the type of probe, and the type of dye. Fluorescently-tagged antibodies or streptavidin are bound to the dye molecule. These secondary components are selected so that they have a strong signal. In a preferred embodiment, prior to imaging all slides are evaluated by a pathologist and regions of interest are identified based on histopathologic and quality criteria including, without excluding others, tumor content, appropriate fixation, necrosis and vascularity.

FISH experiments designed to detect or localize gene expression within cells and tissues rely on the use of a reporter gene, such as one expressing green fluorescent protein (GFP) and the like, to provide the fluorescence signal.

In an alternative technique to interphase or metaphase preparations, fiber FISH, interphase chromosomes are attached to a slide in such a way that they are stretched out in a straight line, rather than being tightly coiled, as in conventional FISH, or adopting a random conformation, as in interphase FISH. This is accomplished by applying mechanical shear along the length of the slide, either to cells that have been fixed to the slide and then lysed, or to a solution of purified DNA. The extended conformation of the chromosomes allows dramatically higher resolution - even down to a few kilobases (kb).

In a further particularly preferred embodiment, parallel to the detection of the polysomy / amplification of the EGFR locus and the LOH level of the PTEN gene, FISH control probes for chromosomes 10 and 7 are used. Those "FISH control probes" are probes that binds specific for the individual chromosomes, thus allowing the determination of the chromosome number. In a preferred embodiment, the FISH control probes are directed to alpha satellite sequences. Alpha satellite sequences, whilst highly repetitive, are specific to each individual chromosome. These sequences flank the centromeres and can present a target measured in megabases. In a preferred embodiment, these FISH control probes would be labeled with different colors than the EGFR and PTEN probes.

In a preferred embodiment, the PTEN FISH probe is a probe that hybridizes to the 10q23 region on chromosome 10 and contains sequences that flank both the 5' and 3' ends of the PTEN gene; in a more preferred embodiment the probe has between 300 and 400 kb. In a more preferred embodiment, the FISH probe for PTEN and the FISH control probe for chromosome 10 are the LSI PTEN (10q23) / CEP 10 dual color probe for PTEN (Vysis, Abbot Molecular) (Goberdhan,D., et al. Human Molecular Genetics 12 (2) (2003): 239-248; Eng, C., et al.Human Mutation 22 (2003): 183-198; Sasaki, H., et al. Am. J. of Pathology 159 (1) (2001): 359-367). Other probes are described by Cairns et al. (Cairns et al. 1997. Cancer Res 57; 4997-5000) and by Hermans et al. (Hermans et al. 2004 Genes Chrom Cancer, 39; 171-184). The LSI PTEN (10q23) is labeled with SpectrumOrange. The CEP 10 SpectrumGreen probe hybridizes to alpha satellite sequences specific to chromosome 10.

In a particular embodiment, the EGFR FISH probe is a probe that hybridizes to the 7p12 region of the chromosome 7 and contains the entire EGFR gene. In a more preferred embodiment, the FISH probe for the EGFR gene and the control probe for chromosome 10 are the LSI EGFR/CEP 7 dual color probe (Vysis, Abbot Molecular). In a particular embodiment the EGFR Probe is labeled with SpectrumOrange and covers an approximately 300 kb region of the 7p12 region of the chromosome 7 (Bredel, M., et al. 1999. Clin Cancer Res 5, 1786-92 ; Harris, A., et al. 1989. J Steroid Biochem 34, 123-31; Kitagawa, Y., et al. 1996. Clin Cancer Res 2, 909-14; Neal, D.E., et al. 1990. Cancer 65, 1619-25 ; Osaki, A., et al. 1992. Am J of Surg 164, 323-6; Pavelic, K., et al. 1993. Anticancer Res 13, 1133-7; Sauter, G., et al. 1996. Am J Pathol 148, 1047-53 ; Torregrosa, D., et al. 1997. Clin Chim Acta 262, 99-119). The CEP 7 probe, labeled with SpectrumGreen, hybridizes to the alpha satellite DNA located at the centromere of chromosome 7 (7p11.1-q11.1).

Other methods known in the arte may be used to determine copy number aberrations; illustrative, non-limitative examples thereof include oligonucleotide-based microarrays (Lucito, et al. 2003. Genome Res. 13:2291-2305; Bignell et al. 2004. Genome Res. 14:287-295; Zhao, et al. 2004. Cancer Research, 64(9):3060-71).

In another particular embodiment, the polysomy / amplification level of the EGFR gene and/or the LOH level of the PTEN gene is measured by using simple sequence length polymorphisms (or microsatelites) (Virmani A.K. et al. Genes chromosomes Cancer 1998, 21 (4) 308-319) or SNPs as genetic markers (Lindblad-toh K et al. Nat. Biotechnol. 2000, 18(9)1001-1005); in a particular embodiment, the polysomy / amplification level of the EGFR gene and/or the LOH level of the PTEN gene is measured by using a SNP array.

In another particular embodiment, the polysomy / amplification level of the EGFR gene and/or the LOH level of the PTEN gene is measured by polynucleotide sequencing, i.e., a method for determining the order of the nucleotide bases in a molecule of a polynucleotide. In a particular embodiment, the polynucleotide sequencing is performed by using a deep sequencing technology. In a more particular embodiment, the sequencing technology uses a large-scale parallel pyrosequencing system capable of sequencing roughly 400-600 megabases of DNA per run with 400-500 base pair read lengths on a suitable sequencer (e.g., Genome Sequencer FLX with GS FLX Titanium series reagents). The system relies on fixing nebulized and adapter-ligated DNA fragments to small DNA-capture beads in a water-in-oil emulsion. The DNA fixed to these beads is then amplified by PCR. Each DNA-bound bead is placed into a well on a PicoTiterPlate, a fiber optic chip. A mix of enzymes such as DNA polymerase, ATP sulfurylase, and luciferase are also packed into the well. The PicoTiterPlate is then placed into the GS FLX System for sequencing. This sequencing technology can sequence any double-stranded DNA and enables a variety of applications including *de novo* whole genome sequencing, re-sequencing of whole genomes and target DNA regions, metagenomics and RNA analysis. In another embodiment, the sequencing technology is the amplicon sequencing technology, i.e., an ultra deep sequencing designed to allow mutations to be detected at extremely low levels, and PCR amplify specific, targeted regions of DNA. This method is used to identify low frequency somatic mutations in cancer samples or discovery of rare variants in HIV infected individuals.

The determination of the level of the polysomy / amplification of EGFR and the level of LOH of PTEN, needs to be correlated with a standard reference value. Said standard reference values are generated by the person skilled in the art in form of a table that divide the patient in ascending number of copies of the EGFR gene or regarding to the level of LOH and ascending number of copies of the PTEN gene.

For EGFR, the reference values can be generated, without limitation, using for example, the classification of Capuzzo et al. (Cappuzzo et al. JNCI 2005;4:643-55). In this classification, the patients are classified into six strata with ascending number of copies of the EGFR gene per cell according to the frequency of tumor cells with specific number of copies of the EGFR gene per chromosome 7. As it was mentioned before, control probes that detect the chromosome 7 for obtaining a ratio number of copies of EGFR gene/chromosome 7 are commercially available. For example, the CEP 7 SpectrumGreen probe (Vysis) that hybridizes to alpha satellite sequences specific to chromosome 7, can be used.

A non limitative example of a table for classifying a patient attending to the polysomy of the EGFR gene is:
1) disomy (D): ≤ 2 copies in >90% of the cells of the sample;
2) low trisomy (LT): ≤ 2 copies in ≥ 40% of the cells of the sample or 3 copies in 10% - 40% of the cells of the sample or ≥ 4 copies in <10% of the cells of the sample;
3) high trisomy (HT): ≤ 2 copies in ≥ 40% of the cells of the sample or 3 copies in ≥ 40% of the cells of the sample or ≥ 4 copies in <10% of the cells of the sample;
4) low polysomy (LP): ≥ 4 copies in 10% - 40% of the cells of the sample; and
5) high polysomy (HP): ≥ 4 copies in ≥ 40% of the cells of the sample or the presence of amplification (presence of tight EGFR gene clusters and a ratio of EGFR gene to chromosome of ≥ 2 or ≥ 15 copies of EGFR per cell in ≥ 10% of analyzed cells of the sample).

As a person skilled in the art will understand, the method [1] of the invention can be performed by using more than one sample of a patient. In such a case, it is considered that exist a "high polysomy level of the EGFR gene" when at least 50%, preferably more than 50%, more than 60%, more than 70%, more than 80%, more than 90%, more than 95%, or even more, of the samples of the patient are classified as "high polysomy" (HP) according to the classification criteria described before.

For the generation of a table for the classification of the patients attending to the level of the LOH and number of copies of the PTEN gene, the person skilled in the art could, for example, classify the patients into 4 strata with ascending number of copies of the PTEN gene per cell according to the frequency of tumor cells with specific number of copies of the PTEN gene per chromosome 10. As it was mentioned before, control probes that detect the chromosome 10 for obtaining a ratio number of copies of PTEN gene/chromosome 10 are commercially available. For example, the CEP 10 SpectrumGreen probe (Vysis) that hybridizes to alpha satellite sequences specific to chromosome 10, can be used.

A non limitative example of a table for classifying a patient attending to the polysomy / level of LOH of the PTEN gene is:
1) disomy (D): 2 copies of each probe in >90% of cells;
2) LOH: <2 copies of PTEN probe in >10% of cells (includes cromosome 10 monosomy or disomy with PTEN LOH, always in >10% cells);
3) polysomy (P): ≥ 3 copies of each probe (PTEN+ CEP 10) in >10% of cells (it does not discriminate between high and low polisomy, or chromosome 10 trisomy); and
4) amplified (AMP): defined by a ratio of the PTEN gene to chromosome 10 of ≥ 2 per cell in ≥ 10% of analyzed cells.

As a person skilled in the art will understand, the method [1] of the invention can be performed by using more than one sample of a patient. In such a case, it is considered that exist a "high LOH level of the PTEN gene" when at least 50%, preferably more than 50%, more than 60%, more than 70%, more than 80%, more than 90%, more than 95%, or even more, of the samples of the patient are classified as "LOH" according to the classification criteria described before.

In the last step of the method [1] of the invention, a high LOH level of the PTEN gene with respect to said standard reference values and high expression levels and/or high polysomy / amplification of the EGFR gene are indicative of a good clinical outcome of the subject.

As a person skilled in the art understand, the samples can also be considered not appropriated for be included in any of the classification strata. Thus, in a preferred embodiment, additionally, samples sections of tissue from the patient are stained with colorants as hematoxylin and eosin (H&E) and reviewed by two or more persons skilled in the art (i.e., pathologists) to assess overall tumor content, necrosis and overall quality (e.g., thermal cautery effect, fixation with morphology, etc). In a preferred embodiment the samples used for determining the expression levels and/or the polysomy levels of the EGFR gene and the LOH level of the PTEN gene have at least 20%, at least 30%, at least 40%, at least 50%, at least 60%, at least 70%, at least 80%, at least 90%, at least 95% of tumor tissue in the sample, and have an appropriate fixation with acceptable morphology.

The teachings of the instant invention do not agree with the conclusions reached by Umesh et al. (Clinical Neuropathology. Vol. 28 - No. 5/2009 (362-372)), who describe a method for predicting the patient outcome of glioma comprising the detection of EGFR amplification and PTEN LOH by immunohistochemistry (IHC) wherein it is stated that EGFR gene amplification associated with LOH of the PTEN gene is a trend to poor survival.

Effectively, as shown in the Example, the teachings of the present invention, which comprises combining EGFR polysomy/amplification (i.e., EGFR expression) with LOH of the PTEN gene wherein the LOH level of the PTEN gene is measured by PCR, a hybridization-based assay, sequencing or SNP arrays [the level of polysomy/amplification of the EGFR gene can be determined at the protein level or, alternatively, at the nucleic acid level] show that an EGFR amplification associated with LOH of the PTEN gene is a trend to good survival.

Assays performed by the inventors have shown that, apparently, the use of IHC to determine the level of the LOH of the PTEN gene is not suitable for accurately assess its relevant expression profile what is especially important in low expressing but not loss of PTEN protein tumors where such a critical difference will allow the true assessment of protein expression in this setting. Further, different results in an IHC assay can be attributed to specific antibodies and their sensitivity for assessing PTEN; there is documented literature to support the challenges for using IHC to assess PTEN when compared with FISH (e.g., Reid et al., British Journal of Cancer 2010, 102:678-684). DNA FISH methods are generally not impacted by the pre-analytic sample preparation, by contrast, such differences in fixation and specimen handling does impact on the PTEN antigen in tissue. One way around this is to use mathematical modeling with quantitative IF to assess true expression vs. Non-specific binding and 'noise' in the system.

Thus, the invention provides method for predicting in a more accurate way the clinical outcome of a subject suffering from glioma.

The findings of the inventors allow the determination of the clinical outcome of a patient suffering glioma measuring the LOH level of the PTEN gene. Thus, it is also disclosed a method for predicting the clinical outcome of a subject suffering from glioma, hereinafter referred to as the method [2], said method comprising:
a) determining the LOH level of the PTEN gene in a sample from the subject, and
b) comparing said LOH level of the PTEN gene with a standard reference value,
wherein the LOH level of the PTEN gene is measured by PCR, or by a hybridization-based assay, or by sequencing, or by a SNP analysis; and
wherein a high LOH level of the PTEN gene with respect to said standard reference value, is indicative of a bad clinical outcome of the subject.

The term glioma has been previously defined. In a preferred embodiment, the glioma is a gliobastoma multiforme (GMB) and more preferably the gliobastoma is an early gliobastoma.

In a preferred embodiment, the clinical outcome is measured as survival.

The term "sample" has been previously defined in relation to the method [1] of the invention and can be applied to any type of biological sample from a patient, such as a biopsy sample, tissue, cell or fluid (whole blood, serum, saliva, semen, sputum, cerebral spinal fluid (CSF), urine, tears, mucus, sweat, milk, brain extracts and the like). In a particular embodiment, said sample is a tumour tissue sample or portion thereof In a more particular embodiment, said tumor tissue sample is a brain tumor tissue sample from a patient suffering from glioma or a formalin embedded brain tissue sample.

The methods for determining the LOH level of the PTEN gene have been described above as well as the standard reference values used. In a preferred embodiment, the LOH level of the PTEN gene is determined by a hybridization-based assay, e.g., by FISH.

The invention also relates to the use of a kit useful in the implementation of the methodology described herein. Thus, in another aspect, the invention relates to the use of a kit comprising a set of agents capable of specifically determining the expression levels and/or the polysomy / amplification of EGFR and the LOH level of the PTEN gene and, optionally, a reagent for detecting a housekeeping gene or the protein encoded by said housekeeping gene and/or a reagent for detecting the chromosomes 7 and 10, for predicting the clinical outcome of a subject suffering from glioblastoma multiforme, wherein the set of agents capable of specifically determining the LOH level of the PTEN gene comprises a pair of oligonucleotide primers suitable for amplifying a specific fragment of the PTEN gene and/or an optionally labeled oligonucleotide probe which selectively binds to a target polynucleotide sequence on the chromosome region of the PTEN gene and/or reagents suitable for performing a sequencing reaction and/or reagents for performing an SNP analysis.

In a particular embodiment of the use of the kit of the invention, the agents of the kit are capable of specifically detecting the mRNA levels of EGFR and/or PTEN genes or the levels of the EGFR and/or PTEN proteins, preferably, the levels of the EGFR protein.

Nucleic acids capable of specifically hybridizing with the EGFR and/or PTEN genes can be one or more pairs of primer oligonucleotides for the specific amplification of fragments of the mRNAs (or of their corresponding cDNAs) of said genes and/or one or more probes for the identification of one or more genes selected from said genes. Nucleic acids capable of specifically hybridizing with the EGFR and/or PTEN genes can be as well FISH probes.

Antibodies, or a fragment thereof, capable of detecting an antigen, capable of specifically binding to EGFR and/or PTEN proteins or to variants thereof are, for example, monoclonal and polyclonal antibodies, antibody fragments, Fv, Fab, Fab' y F(ab')2, ScFv, diabodies, triabodies, tetrabodies and humanised antibodies.

Said reagents, specifically, the probes and the antibodies, may be fixed onto a solid support, such as a membrane, a plastic or a glass, optionally treated in order to facilitate fixation of said probes or antibodies onto the support. Said solid support, which comprises, at least, a set of antibodies capable of specifically binding to EGFR and/or PTEN proteins or to variants thereof, and/or probes specifically hybridized with the EGFR and/or PTEN genes, may be used for the detection of the expression levels by means of the array technology.

The kits for use of the invention optionally comprise additional reagents for detecting a polypeptide encoded by a housekeeping gene or the mRNA encoded by said housekeeping genes. The availability of said additional reagent allows the normalization of measurements taken in different samples (e.g. the test sample and the control sample) to exclude that the differences in expression of the different biomarkers are due to a different amount of total protein in the sample rather than to real differences in relative expression levels. Housekeeping genes, as used herein, relates to genes which code for proteins which are constitutively expressed and carry out essential cellular functions. Preferred housekeeping genes for use in the present invention include β-2-microglobulin, ubiquitin, 18-S ribosomal protein, cyclophilin, GAPDH and actin.

In an embodiment, the kit for use of the invention may contain reagents suitable for performing a sequencing reaction and/or reagents for performing an SNP array, e.g., enzymes, nucleotides, etc.

In another embodiment, the invention relates to the use of a kit of the invention for predicting the clinical outcome of a subject suffering from gliobastoma multiforme, wherein if said agents detect a high expression level and/or high level of polysomy / amplification of the EGFR gene and a high LOH level of the PTEN gene, with respect to standard reference values, in a sample from said subject, then the clinical outcome of the subject is good.

Methods for detecting the expression levels of EGFR and the methods for determining the polysomy of the EGFR gene and the LOH level of the PTEN gene as well as the standard reference values have been described previously (see, for example, method [1] of the invention).

In another embodiment, it is also disclosed the use of the EGFR expression levels and/or polysomy / amplification levels of the EGFR gene and the LOH level of the PTEN gene as predictive marker of the clinical outcome of a glioma patient. In a particular embodiment, the glioma is a gliobastoma multiforme (GMB). In another particular embodiment, the clinical outcome is measure as survival.

In another particular embodiment, it is also disclosed the use of erlotinib and/or temozolomide in the manufacture of a medicament for the treatment of a glioma in a subject suffering from a glioma, wherein the medicament is for a subject having a high LOH level of the PTEN gene, as measured by PCR, by a hybridization-based assay, by sequencing or by a SNP analysis, with respect to a standard reference value and high expression levels and/or high polysomy / amplification of the EGFR gene with respect to standard reference values. Alternatively, it is also disclosed erlotinib and/or temozolomide for use in the treatment of a glioma in a subject suffering from a glioma, wherein the medicament is for a subject having a high LOH level of the PTEN gene, as measured by PCR, by a hybridization-based assay, by sequencing or by a SNP array, with respect to a standard reference value and high expression levels and/or high polysomy / amplification of the EGFR gene with respect to standard reference values. The glioma may be a gliobastoma multiforme (GMB). The clinical outcome may be measured as survival.

In another embodiment, it is also disclosed the use of radiotherapy in a regime for the treatment of a glioma in a subject suffering from a glioma, wherein said subject has a high LOH level of the PTEN gene, as measured by PCR, by a hybridization-based assay, by sequencing or by a SNP analysis, with respect to a standard reference value and high expression levels and/or high polysomy / amplification of the EGFR gene with respect to standard reference values. The glioma may be a gliobastoma multiforme (GMB). The clinical outcome may be measured as survival.

### EXAMPLES

### I. MATERIAL AND METHODS

### Patients and specimens

Multiple formalin-fixed, paraffin-embedded blocks from fifty-six (56) primary brain tumor specimens were obtained from the Hospital Universitari de Belvitge. These patients were part of a longitudinal cohort and selected utilizing pre-determined inclusion criteria which included a primary diagnosis of any one of the following criteria: astrocytic, oligodendrocytic and glial tumor, with continuous follow-up for a median of 3 years and available tissue material. Clinical data was abstracted from the patient's clinical records utilizing a set of pre-determined clinical-pathologic and outcome data fields (see Table 1). The histologic breakdown of the tumor specimens were as follows:
I. Astrocytoma group, total 44 patients: pilocytic (1), diffuse (13), anaplastic (16), and glioblastoma multiforme (14);
II. Oligodendroglioma group, total 6 patients: oligodendroglioma (6); and
III. Mixed, total 4 patients: oligoastrocytoma (3), and anaplastic oligoastrocytoma (1).

A comprehensive review of the available clinical data files was performed on all patients in which formalin-fixed, paraffin-embedded (FFPE) samples/blocks were available for further analysis.

5 µm sections were obtained from all blocks, stained with hematoxylin and eosin (H&E) and reviewed by two pathologists to assess overall tumor content (≥ 50% tumor in at least one 200X field), necrosis and overall quality (e.g., thermal cautery effect, appropriate fixation with acceptable morphology, etc). Subsequent sections were then obtained for DNA FISH assays and quantitative immunofluorescence (IF) as outlined below. All blocks were retained for subsequent studies including RT-PCR.

| **Table 1. Clinical data fields for entire cohort with breakdown by diagnostic group** | | **Glioblastoma** | **Astrocytoma** | **Mixed/others** | **Non available** |
|---|---|---|---|---|---|
| | | N=15 | N=26 | N=5 | N=10 |
| Patients gender: | Male | 8 | 17 | 4 | 5 |
| | Female | 7 | 9 | 1 | 3 |
| | Non available | 0 | 0 | 0 | 2 |
| Patients race | caucasian | 15 | 25 | 5 | 8 |
| | Non available | 0 | 1 | 0 | 2 |
| Symptoms seizures | Yes | 4 | 13 | 2 | 5 |
| | No | 11 | 13 | 2 | 3 |
| | Non available | 0 | 0 | 1 | 2 |
| Symptoms high intracranial pressure | Yes | 7 | 5 | 1 | 1 |
| | No | 8 | 21 | 3 | 7 |
| | Non available | 0 | 0 | 1 | 2 |
| Number of lesions in pretreatment | Image 1 | 12 | 21 | 5 | 7 |
| | Image 2 | 1 | 1 | 0 | 0 |
| | multiples | 2 | 3 | 0 | 1 |
| | Non available | 0 | 1 | 0 | 2 |
| 1 location of lesions in pretreatment image: | Frontal lobe | 10 | 10 | 3 | 5 |
| | Temporal lobe | 3 | 3 | 0 | 1 |
| | Parietal lobe | 1 | 6 | 1 | 0 |
| | Operculum | 0 | 1 | 0 | 0 |
| | Brain stem | 1 | 2 | 1 | 0 |
| | Spinal cord | 0 | 1 | 0 | 0 |
| | Optic nerve | 0 | 0 | 0 | 1 |
| | Interhemispheric cisure | 0 | 1 | 0 | 0 |
| | Cerebellum | 0 | 0 | 0 | 1 |
| | | 0 | 1 | 0 | 0 |
| | Non available | 0 | 1 | 0 | 2 |
| 2 location of lesions in pretreatment image | Temporal lobe | 1 | 1 | 0 | 1 |
| | Parietal lobe | 3 | 1 | 0 | 0 |
| | Occipital lobe | 2 | 1 | 0 | 1 |
| | Corpus callosum | 0 | 1 | 0 | 0 |
| | Basal ganglia | 2 | 0 | 0 | 0 |
| | Brain stem | 0 | 1 | 0 | 0 |
| | Thalamus | 0 | 0 | 0 | 1 |
| | Non available | 7 | 21 | 5 | 7 |
| 1 side of lesions in pretreatment image | Right | 9 | 12 | 2 | 6 |
| | Left | 4 | 11 | 3 | 2 |
| | Unknown | 0 | 1 | 0 | 0 |
| | Non available | 2 | 2 | 0 | 2 |
| 2 side of lesions in pretreatment image | Left | 1 | 1 | 0 | 1 |
| | Non available | 14 | 25 | 5 | 9 |
| Type of specimen | Biopsy | 1 | 6 | 0 | 4 |
| | Stereotaxic biopsy | 2 | 4 | 0 | 1 |
| | Resection | 12 | 16 | 5 | 3 |
| | Non available | 0 | 0 | 0 | 2 |
| Type of postsurgical removal | Complete | 4 | 11 | 1 | 6 |
| | Incomplete | 10 | 12 | 4 | 2 |
| | Non available | 1 | 3 | 0 | 2 |
| Radiation therapy | Yes | 7 | 12 | 3 | 1 |
| | No | 8 | 12 | 2 | 7 |
| | Non available | 0 | 2 | 0 | 2 |
| Type of radiation | Focal | 5 | 8 | 0 | 1 |
| | Non available | 10 | 18 | 5 | 9 |
| Total dose of radiation (**) (Available data: n=20) | | 60.0 (60.0-60.0) | 60.0 (40.0-60.0) | 51.0 (48.0-54.0) | 60.0 (60.0-60.0) |
| Chemotherapy | Yes | 2 | 9 | 1 | 1 |
| | No | 13 | 15 | 4 | 7 |
| | Non available | 0 | 2 | 0 | 2 |
| Type of first chemotherapy | BCNU | 0 | 7 | 0 | 0 |
| | TMZ | 2 | 1 | 0 | 0 |
| | PCV | 0 | 1 | 0 | 1 |
| | Non available | 13 | 17 | 5 | 9 |
| Recurrence or progression | Yes | 4 | 13 | 4 | 6 |
| | No | 9 | 10 | 1 | 1 |
| | Non available | 2 | 3 | 0 | 3 |
| Type of first recurrence | Focal | 3 | 8 | 4 | 6 |
| | Diffuse | 1 | 1 | 0 | 0 |
| | Multiple | 0 | 2 | 0 | 0 |
| | Non available | 11 | 15 | 1 | 4 |
| Treatment of first recurrence | Yes | 3 | 10 | 4 | 5 |
| | No | 1 | 1 | 0 | 1 |
| | Non available | 11 | 15 | 1 | 4 |
| 1 type of treatment of first recurrence | Radiation | 0 | 2 | 1 | 1 |
| | Chemotherapy | 2 | 3 | 1 | 0 |
| | Surgery | 1 | 4 | 2 | 4 |
| | Radiosurgery | 0 | 1 | 0 | 0 |
| | Non available | 12 | 16 | 1 | 5 |
| 2 type of treatment of first recurrence | Radiation | 0 | 2 | 0 | 4 |
| | Chemotherapy | 0 | 1 | 0 | 0 |
| | Non available | 15 | 23 | 5 | 6 |
| 3 type of treatment of first recurrence | Chemotherapy | 0 | 1 | 0 | 1 |
| | Non available | 15 | 25 | 5 | 9 |
| Karnovsky value pretreatment (**) (Available data: n=49) | | 70.0(40.0-100) | 90.0 (20.0-100) | 95.0 (90.0-100) | 100(60.0 100) |
| Karnovsky value postreatment (**) (Available data: n=46) | | 60.0(30.0-90.0) | 80.0(10.0-100) | 95.0 (80.0-100) | 100(30.0 100) |
| Death of patient | Yes | 15 | 15 | 1 | 1 |
| | No | 0 | 7 | 3 | 5 |
| | Non available | 0 | 4 | 1 | 4 |

### DNA EGFR and PTEN FISH

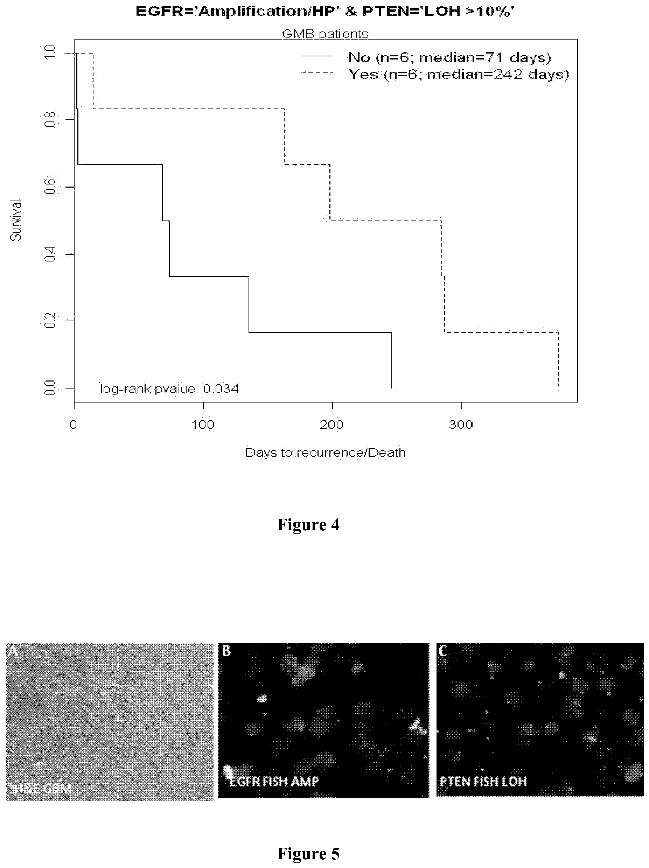

Using the LSI EGFR/CEP 7 dual color probe (Vysis, Abbot Molecular) for EGFR and the LSI PTEN (10q23)/CEP 10 dual color probe for PTEN (Vysis, Abbot Molecular), DNA FISH including hybridization, washing and fluorescence detection was performed on all specimens in the cohort as per standard protocols (Smith et al., 2001. JNCI; 93:1246-1256). Briefly, paraffin sections were dewaxed in xylenes, microwaved in 10 mmol/L sodium citrate (pH 6.0) solution for 5-10 minutes, cooled to room temperature, rinsed, and then treated with pepsin HCl for 5 minutes at 37°C before being rinsed and dehydrated. The prewarmed probe mixture was applied to the slides, and a cover-slip sealed in place with rubber cement. The slides were then denatured at 85°C for 4 to 6 minutes using an automated hybridization chamber and then incubated overnight at 37°C. After standard post-hybridization washes the slides were stained with the DNA stain DAPI and mounted with antifade (Vectashield). Prior to imaging all H&E slides were evaluated by a pathologist and regions of interest were identified based on histopathologic and quality criteria including tumor content, appropriate fixation, necrosis and vascularity. A H&E image of individual GBM cases is illustrated in Figure 5A. All slides were imaged using a Nikon immunofluorescent microscope by a trained scientist blinded to outcome. Criteria for FISH anomalies were defined by use of histologically normal brain specimens. Simple gain required 10% or more of nuclei with three or more locus-specific probe signals. Loss of the q arm of chromosome 10 required the overall mean PTEN/CEP10 ratio to be less than 0.90. Amplification was applied for both EGFR and PTEN and required that the ratio must be 2 or more and that 10% or more of nuclei had more than three EGFR and or PTEN signals. Representative regions of interest were acquired for documentation of signal in all specimens (Examples of representative images of FISH AMP/HP of EGFR and monosomy of PTEN are shown in Figure 5B and C, respectively). For EGFR FISH we followed the Capuzzo et al. (Cappuzzo F, et al., JNCI 2005;4: 643-55) classification scheme and identified 6 categories for EGFR status in all tumor specimens. These individual states included: D=disomy, HT=high trisomy, LT=low trisomy, HP=high polysomy, LP=low polysomy, and AMP=amplification. In this schema both HP and AMP together are considered amplification. PTEN status was characterized as D=disomy, LOH=>10%, P=polysomy, AMP=amplified and NE=not evaluable. All cases were reviewed by a single observer and recorded anonymously with respect to tumor type or outcome.

### Quantitative Immunofluorescence (IF)

Utilizing previous established methods (Cordon Cardo et al., 2007 JCI 2007; 117; 1876-83; Donovan et al., 2008 JCO;26:3923-3929) the multiplex-1 (mplex1) assay was constructed incorporating antibodies to: GFAP (glial fibrillary acidic protein), PTEN (phosphatase and tensin homolog), pAKT and Ki67 combined with the nuclear stain DAPI (4',6-diamidino-2-phenylindole) - see Table 2 for a complete review of antibodies used within the mplex1 assay. The individual antibodies were evaluated in simplex IF assays using control tissues and cell lines to confirm expression sensitivity and specificity prior to inclusion in the multiplex-1 assay.

**Table 2. Reagent list**

| **Antibody** | **Vendor** | **Catalog** # | **Dilution** | **Isotype** | **Labels** | **Samples** |
|---|---|---|---|---|---|---|
| | | | 1:30 | M IgG1 | 2X M 488 | GBM cases, NL brain LNCaP |
| PTEN | Neomarkers | | | | | Tonsil |
| Ki67 | Dako | | 1:100 | M IgG1 | 2X M 555 | Same |
| pAKT | CST | #3787 | 1:25 | R IgG | 2X R 594 | Same |
| GFAP | Dako | | 1:200 | M IgG1 | 2X M 647 | Same |

In brief, FFPE samples were de-paraffinized, rehydrated and subjected to an antigen retrieval process with the Reveal buffer system (Biocare Medical). A series of four antibodies were combined with DAPI into a multiplex immunofluorescent assay. The reagents listed in Table 2 were differentially labeled and with the identified fluorochromea and then sequentially applied onto individual sections using a Nemesis 7200 immunostainer (BioCare Medical). A minimum of three images or regions of interest (ROI) were acquired by a pathologist (blinded to outcome) using a Nikon 90i immunofluorescent microscope equipped with a CRI spectral imaging system (CRI). Utilizing pre-developed unmixing libraries and CRI software, individual gray scale images were generated which represent the antibody - filter combination under investigation. The individual images were evaluated for signal:noise ratio, cellular distribution and co-distribution with other antibody-fluorochrome labeled reagents. Using the existing CRI software manual thresholds were created for individual antibody-fluorochrome combinations to maximize signal:noise and preserving distribution. Quantitative metrics were generated using the software to identify percentages of individual cell populations exhibiting a positive signal (based on the threshold), normalized to the tumor region under evaluation. A series of prostate cancer cell lines including LNCaP, PC3 and DU145 (ATCC) were obtained, grown to confluency, harvested as agar cell pellets, fixed and embedded in paraffin and then all three cell lines were placed into a cell array for quality control both during assay development and investigation of brain tumor cases.

### Results and Discussion

### Patients and specimens

There were 54 patients with available clinical records and FFPE tissue samples appropriate for evaluation. The breakdown of patients is listed in Table 3 with the majority of patients in the astrocytoma diagnostic category. The individual demographic features including sex, location of mass, treatment and outcome data are outlined in detail in Table 1.

**Table 3. Diagnosis category of the patients**

| **Original codes** | **Pure GBM** | **Astrocytoma** | **Oligodendroglioma** | **Mixed/other** |
|---|---|---|---|---|
| 001: astrocytoma grade II | 0 | 10 | 0 | 0 |
| 002: astrocytoma grade III | 0 | 16 | 0 | 0 |
| 003: glioblastoma | 15 | 0 | 0 | 0 |
| 004: oligoastrocytoma grade II | 0 | 0 | 0 | 3 |
| 005: oligoastrocytoma grade III | 0 | 0 | 0 | 2 |
| 006: oligodendroglioma grade II | 0 | 0 | 4 | 0 |
| 007: oligodendroglioma grade III | 0 | 0 | 1 | 0 |
| 008: pilocytic astrocitoma | 0 | 2 | 0 | 0 |
| 009: gliomatosis cerebri | 0 | 0 | 0 | 1 |
| 999: Non available | 0 | 0 | 0 | 2 |

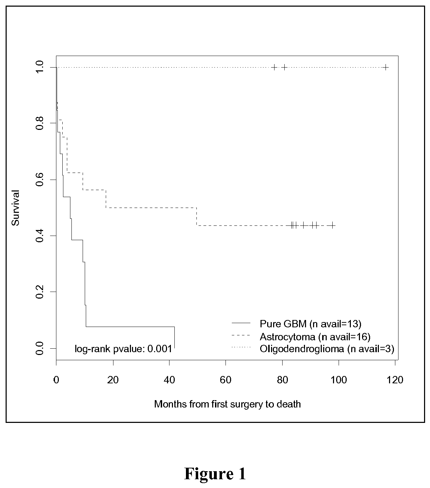

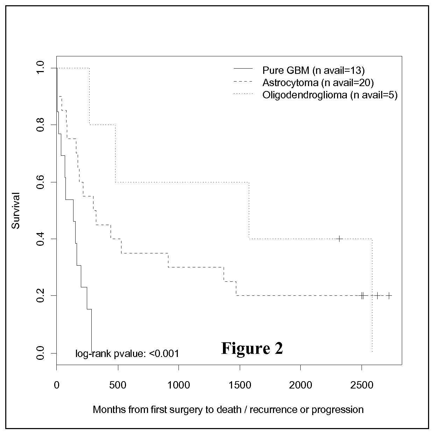

In order to understand general survival trends within the cohort and in particular the glioblastoma group vs. others we performed Kaplan-Meier survival function analyses to estimate survival between the three groups. Figure 1 is the Kaplan-Meier survival curve demonstrating reduced survival in the glioblastoma group vs. the anaplastic astrocytoma and mixed categories (log-rank test P=0.001). Figure 2 is a second Kaplan-Meier survival function curve which evaluates a more composite end-point which includes progression free and overall survival in the same three diagnostic categories (log-rank test P<0.001). In agreement with the literature, the glioblastoma group has a reduced overall and progression free survival when compared to the astrocytoma and pure oligodendroglioma group which has the longest survival time of all three diagnostic categories. As evident from the curves, a subset of the anaplastic astrocytoma group appears to behave like the glioblastoma category.

### EGFR and PTEN FISH

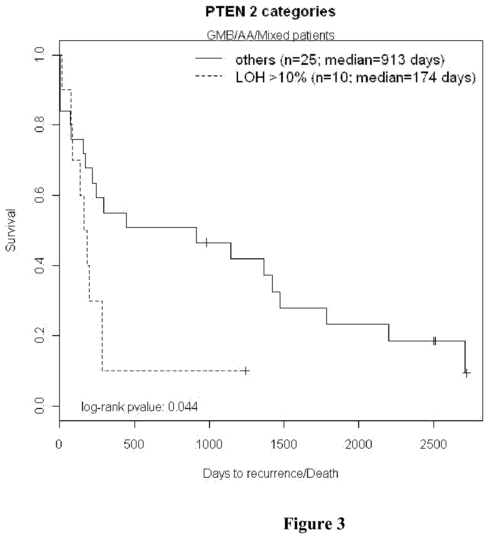

Given the frequency of EGFR amplification (approximately 40%) and reported role that EGFR plays in the development of glioblastoma, the first DNA FISH study performed was on the characterization of EGFR. Using the scoring methods of both Cappuzzo et al. (Capuzzo et al., 2005 JNCI; 4:643-55) and Smith et al. (Smith et al., 2001 JNCI; 93:1246-1256), we investigated EGFR profiles in all brain tumor cases in the group. Given the overall cohort size we opted to further classify patients as positive if they exhibited either polysomy and/or amplification of the EGFR locus or chromosome 7. This is in contrast to some reported studies which independently characterize EGFR DNA FISH as either polysomy or amplification. In our study 71 % of the GBM patients had EGFR polysomy/amplification compared with 45% in the anaplastic astrocytoma group. We did not find EGFR amplification or polysomy to be associated with survival when examining the entire cohort or individual subgroups (all patients log-rank test P=0.8; glioblastoma only patients P=0.1). Dual-color FISH was also performed with probes for PTEN utilizing a similar scoring system as EGFR; however in addition to disomy and polysomy we included the loss of PTEN as higher than 10% in tumor epithelial nuclei. In our studies 61% of GBM samples had PTEN LOH compared with 25% in the anaplastic astrocytoma group. There is sparse literature on the evaluation of PTEN by DNA FISH in brain tumor samples with most studies using more molecular techniques such as sequencing, RT-PCR and immunohistochemistry (IHC). Noteworthy in our cohort, PTEN LOH was univariately and statistically associated with reduced survival in the combined glioblastoma multiforme, astrocytoma and mixed patient group (n=25 without LOH, median survival 913D vs. n=10 with LOH, median survival 174D; log-rank test p=0.04) [see Figure 3].

Neither EGFR polysomy/amplification or PTEN loss were significant, independent predictors for survival in the glioblastoma only group (P=0.1); however, when combined we observed a trend towards significance with an increase in overall survival (n=6 with both EGFR/PTEN, median survival 242D vs. n=6 without, median survival 71D; log-rank test P=0.034) (Figure 4). The hypothesis is that the combination of PTEN LOH and EGFR amplification in glioblastoma may represent a subset of patients with a tumor phenotype more amenable to radiation and/or temozolomide treatment.

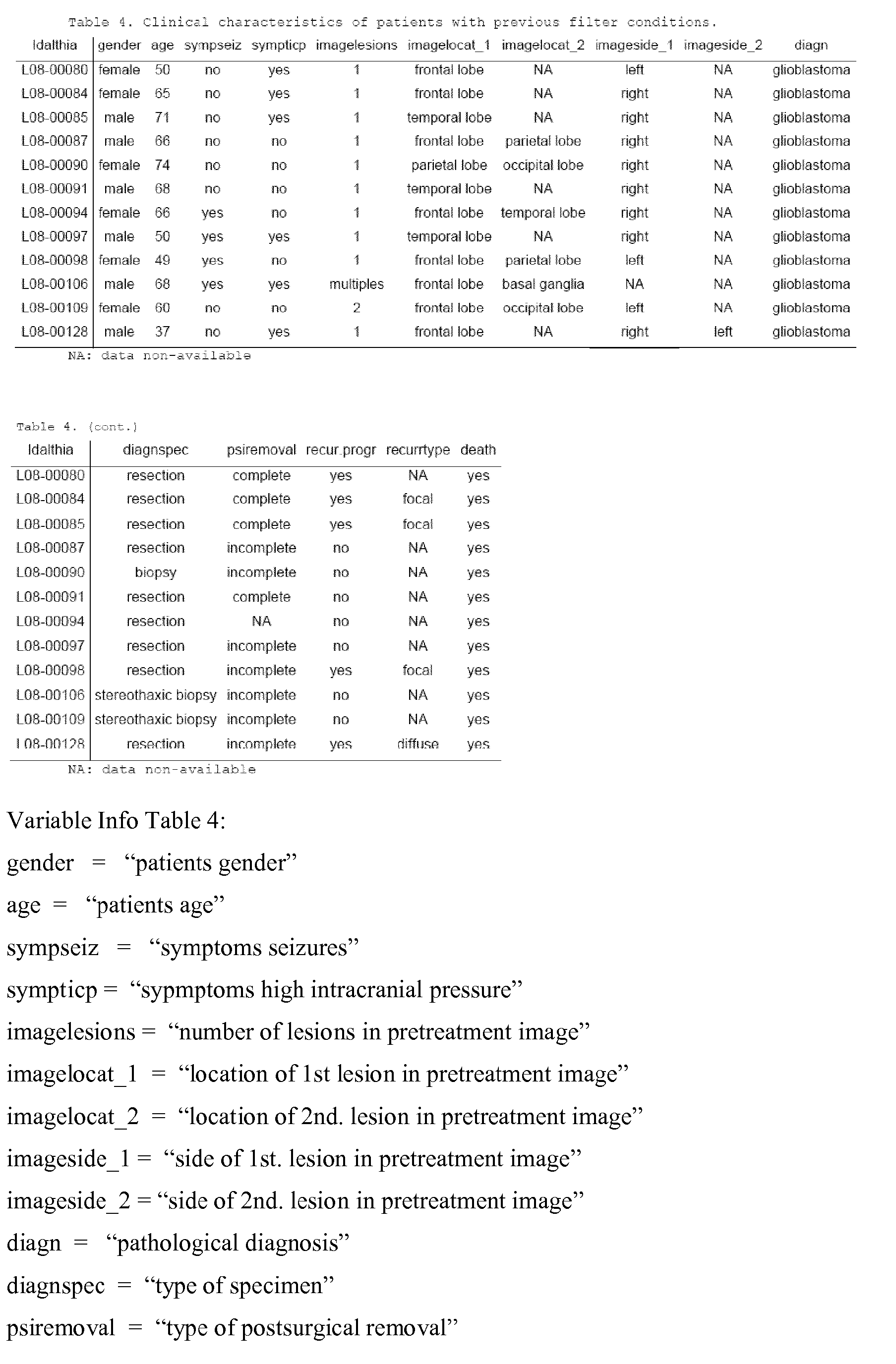

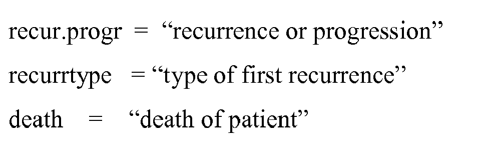

In Table 4, 12 patients were included, 6 with the EGFR AMP / PTEN LOH phenotype and 6 without (see column 2), EGFR/PTEN status (yes / no with respect to phenotype). Of the 6 Glioblastoma multiforme (GBM) patients with this phenotype, 3 exhibited no evidence of clinical recurrence. The 3 patients who did recur in the EGFR AMP / PTEN LOH positive group had received radiation and one received both radiation and the chemotherapeutic agent temozolomide (TMZ). Although the number of patients is small the data suggest that GBM patients with a tumor exhibiting the EGFR AMP / PTEN LOH have a better overall clinical outcome than patients without this phenotype regardless of current treatment including radiation and chemotherapy. We conclude that the identified phenotype which involves both EGFR signaling and PTEN loss may reflect an overall good acting GBM, irrespective of current treatment modalities and that newer investigative therapies and clinical trials would benefit from focusing on the intersect of these two pathways to further improve outcome.

## Claims

1. A method for predicting the clinical outcome of a subject suffering from glioblastoma multiforme (GBM) that comprises:
a) determining the expression level or the polysomy/amplification level of the EGFR gene and the loss of heterozygosity (LOH) level of the PTEN gene in a sample from the same subject, and
b) comparing said expression level or the polysomy/amplification level of the EGFR gene and the LOH level of the PTEN gene with standard reference values,
wherein the LOH level of the PTEN gene is measured by PCR, by a hybridization-based assay, by sequencing technology, or by a SNP analysis; and
wherein a high LOH level of the PTEN gene with respect to said standard reference value and a high expression level and/or high level of polysomy/amplification of the EGFR gene with respect to said standard reference values are indicative of a good clinical outcome of the subject.

2. Method according to claim 1, wherein the clinical outcome is measured as survival.

3. Method according to anyone of claims 1 or 2, wherein the sample is a cell or tissue sample, preferably a tumor tissue sample.

4. Method according to anyone of claims 1 to 3, wherein the expression level of the EGFR gene is measured by determining the mRNA and/or protein expression level of said gene.

5. Method according to claim 1, wherein said hybridization-based assay comprises a Southern blot, *in situ* hybridization (ISH), fluorescence *in situ* hybridization (ISH), or a comparative genomic hybridization (CGH) assay.

6. Method according to claim 1 and 5, wherein the LOH level of the PTEN gene is determined by FISH.

7. Method according to anyone of claims 1 to 6, wherein the glioblastoma is early glioblastoma.

8. Use of a kit comprising a set of agents capable of specifically determining the expression level or the polysomy/amplification level of the EGFR gene and the LOH level of the PTEN gene for predicting the clinical outcome of a subject suffering from glioblastoma multiforme, wherein the set of agents capable of specifically determining the LOH level of the PTEN gene comprises a pair of oligonucleotide primers for amplifying a specific fragment of the PTEN gene, or an optionally labeled oligonucleotide probe which selectively binds to a target polynucleotide sequence on the chromosome region of the PTEN gene, or reagents suitable for performing a sequencing reaction, or reagents for performing an SNP analysis, and wherein if said agents detect a high expression levels or a high level of polysomy/amplification of the EGFR gene and a high LOH level of the PTEN gene, with respect to reference values, then the clinical outcome of the subject is good.

9. Use according to claim 8, wherein the kit further comprises a reagent for detecting a housekeeping gene or the protein encoded by said housekeeping gene.

10. Use according to any of claims 8 or 9, wherein the set of agents for specifically determining the expression level and/or the level of polysomy / amplification of the EGFR comprises a reagent for detecting the mRNA level of the EGFR gene or the level of the EGFR protein.

11. Use according to claim 10, wherein the reagent for detecting the mRNA level of the EGFR gene or the level of the EGFR protein comprises a probe that specifically hybridizes with the EGFR gene, or an antibody that binds to the EGFR protein.

## Patentansprüche

1. Verfahren zur Vorhersage des klinischen Ergebnisses eines am Glioblastoma multiforme (GBM) erkrankten Subjekts, umfassend:
a) Bestimmen des Expressionslevels oder des
Polysomie/Amplifikationslevels des EGFR-Gens, und des Verlusts der Heterozygotie (LOH) Levels des PTEN- Gens in einer Probe des selben Subjekts, und
b) Vergleichen dieses Expressionslevels oder des
Polysomie/Amplifikationslevels des EGFR-Gens und des LOH Levels des PTEN-Gens mit Standardreferenzwerten,
wobei das LOH Level des PTEN-Gens per PCR, durch einen Hybridisierungstest, durch Sequenziertechnologie oder durch SNP-Analyse gemessen wird, und
wobei ein hoher LOH Level des PTEN-Gens in Bezug auf diesen Standardreferenzwert und ein hohes Expressionslevel und/oder hohes Level der Polysomie/Amplifikation des EGFR-Gens in Bezug auf diese Standardreferenzwerte, indikativ für ein gutes klinisches Ergebnis des Subjekts sind.

2. Verfahren gemäß Anspruch 1, wobei das klinische Ergebnis als Überleben gemessen wird.

3. Verfahren nach einem der Ansprüche 1 oder 2, wobei die Probe eine Zellprobe oder Gewebeprobe, vorzugsweise eine Tumorgewebeprobe, ist.

4. Verfahren nach einem der Ansprüche 1 bis 3, wobei das Expressionslevel des EGFR-Gens durch Bestimmen der mRNA und/oder des Proteinexpressionslevels des Gens gemessen wird.

5. Verfahren gemäß Anspruch 1, wobei der Hybridisierungstest einen Southern Blot, *In-Situ*-Hybridisierung (ISH), Fluoreszenz-*In-Situ*-Hybridisierung (FISH), oder vergleichende genomische Hybridisierung (CGH) umfasst.

6. Verfahren gemäß Anspruch 1 und 5, wobei das LOH Level des PTEN-Gens durch FISH bestimmt wird.

7. Verfahren gemäß einem der Ansprüche 1 bis 6, wobei das Glioblastom ein frühes Glioblastom ist.

8. Verwendung eines Kits, umfassend ein Set an Agenzien, die geeignet zur spezifischen Bestimmung des Expressionslevels oder des Polysomie-/Amplifikationslevels des EGFR-Gens und des LOH Levels des PTEN-Gens sind, zur Vorhersage des klinischen Ergebnisses eines an Glioblastoma multiforme erkrankten Subjekts, wobei das zur spezifischen Bestimmung des LOH Levels des PTEN-Gens geeignete Set an Agenzien ein Paar Oligonukleotid-Primer zur Amplifikation eines spezifischen Fragments des PTEN-Gens oder eine optional markierte Oligonukleotid-Sonde, welche selektiv eine Ziel-Polynukleotidsequenz innerhalb der chromosomalen Region des PTEN-Gens bindet oder geeignete Reagenzien zur Durchführung einer Sequenzierungsreaktion oder Reagenzien zur Durchführung einer SNP-Analyse enthält, und wobei bei Detektion eines, in Bezug auf Referenzwerte hohen Expressionslevels oder eines hohen Polysomie-/Amplifikationslevels des EGFR-Gens und eines hohen LOH Levels des PTEN-Gens durch diese Reagenzien das klinische Ergebnis des Subjekts gut ist.

9. Verwendung gemäß Anspruch 8, wobei das Kit ferner ein Reagenz zur Detektion eines Housekeeping-Gens oder des durch das Housekeeping-Gen codierten Proteins enthält.

10. Verwendung gemäß einem der Ansprüche 8 oder 9, wobei das Set an Agenzien zur spezifischen Bestimmung des Expressionslevels und/oder des Polysomie-/Amplifikationslevels des EGFR-Gens, ein Reagenz zur Detektion des mRNA Levels des EGFR-Gens oder des EGFR-Proteinlevels enthält.

11. Verwendung gemäß Anspruch 10, wobei das Reagenz zur Detektion des mRNA Levels des EGFR-Gens oder des EGFR-Proteinlevels eine spezifisch mit dem EGFR-Gen hybridisierende Sonde oder einen Antikörper, der an das EGFR-Protein bindet, umfasst.

## Revendications

1. Un procédé de prédiction du résultat clinique d'un sujet atteint d'un glioblastome multiforme (GBM) qui comprend :
a) la détermination du taux d'expression ou du taux de polysomie/amplification du gène EGFR et le taux de perte d'hétérozygotie (LOH) du gène PTEN dans un échantillon provenant du même sujet, et
b) la comparaison dudit taux d'expression ou du taux de polysomie/amplification du gène EGFR et le taux de LOH du gène PTEN par rapport à des valeurs de référence normales,
où le taux de LOH du gène PTEN est mesuré par PCR, par un essai fondé sur une hybridation, par technologie de séquençage, ou par une analyse SNP ; et
où un taux élevé de LOH du gène PTEN par rapport à ladite valeur de référence normale et un taux élevé d'expression et/ou un taux élevé de polysomie/amplification du gène EGFR par rapport auxdites valeurs de référence normales sont indicatifs d'un bon résultat clinique du sujet.

2. Procédé selon la revendication 1, où le résultat clinique est mesuré en tant que survie.

3. Procédé selon la revendication 1 ou 2, où l'échantillon est un échantillon cellulaire ou tissulaire, de préférence un échantillon de tissu tumoral.

4. Procédé selon l'une quelconque des revendications 1 à 3, où le taux d'expression du gène EGFR est mesuré en déterminant le taux d'expression ARNm et/ou protéique dudit gène.

5. Procédé selon la revendication 1, où ledit essai fondé sur une hybridation comprend un Southern blot, une hybridation *in situ* (ISH), une hybridation *in situ* en fluorescence (FISH), ou un essai d'hybridation génomique comparative (CGH).

6. Procédé selon les revendications 1 et 5, où le taux de LOH du gène PTEN est déterminé par FISH.

7. Procédé selon l'une quelconque des revendications 1 à 6, où le glioblastome est un glioblastome précoce.

8. Utilisation d'un kit comprenant un ensemble d'agents capables de déterminer spécifiquement le taux d'expression ou le taux de polysomie/amplification du gène EGFR et le taux de LOH du gène PTEN afin de prédire le résultat clinique d'un sujet atteint d'un glioblastome multiforme, où l'ensemble d'agents capables de déterminer spécifiquement le taux de LOH du gène PTEN comprend une paire d'amorces oligonucléotidiques permettant d'amplifier un fragment spécifique du gène PTEN, ou une sonde oligonucléotidique éventuellement marquée qui se lie sélectivement à une séquence polynucléotidique cible sur la région chromosomique du gène PTEN, ou des réactifs aptes à effectuer une réaction de séquençage, ou des réactifs aptes à effectuer une analyse SNP, et où si lesdits agents détectent un taux élevé d'expression ou un taux élevé de polysomie/amplification du gène EGFR et un taux élevé de LOH du gène PTEN, par rapport à des valeurs de référence, alors le résultat clinique du sujet est bon.

9. Utilisation selon la revendication 8, où le kit comprend en outre un réactif permettant de détecter un gène domestique ou la protéine codée par ledit gène domestique.

10. Utilisation selon la revendication 8 ou 9, ou l'ensemble d'agents permettant de déterminer spécifiquement le taux d'expression et/ou le taux de polysomie/amplification du gène EGFR comprend un réactif permettant de détecter le taux d'ARNm du gène EGFR ou le taux de la protéine EGFR.

11. Utilisation selon la revendication 10, où le réactif permettant de détecter le taux d'ARNm du gène EGFR ou le taux de protéine EGFR comprend une sonde qui s'hybride spécifiquement avec le gène EGFR, ou un anticorps qui se lie à la protéine EGFR.
